# EUROPEAN PATENT APPLICATION

(11) **EP 1 475 380 A1**
(43) Date of publication of application: **10.11.2004**
(21) Application number: 03701109.5
(22) Date of filing: 17.01.2003
(51) Int. Cl.: C07D 471/04, C07D 471/22, C09K 11/06, H05B 33/14

(54) **WHITE LIGHT EMITTING COMPOUND, WHITE LIGHT EMISSION ILLUMINATOR, AND WHITE LIGHT EMISSION ORGANIC EL DEVICE**

(30) Priority: 18.01.2002 JP 2002010895; 21.01.2002 JP 2002012223
(71) Applicant: HIROSE ENGINEERING CO., LTD., Tokyo 146-0092 (JP)
(72) Inventor: NAKAYA, Tadao c/o Hirose Engine. Co., Ltd., Ohta-ku, Tokyo 146-0092 (JP); IKEDA, Atsushi c/o Hirose Engine. Co., Ltd., Ohta-ku, Tokyo 146-0092 (JP); SAIKAWA, Tomoyuki c/o Hirose Engine.Co.,Ltd., Ohta-ku, Tokyo 146-0092 (JP); ETOH, Naonobu c/o Hirose Engine. Co., Ltd., Ohta-ku, Tokyo 146-0092 (JP); KIMURA, Yoshihiro 304, Fujinodaidanchi 1-48, Tokyo 194-0032 (JP); YAMAUCHI, Takao 13-21, Kitaterao 1-chome, Kanagawa 230-0074 (JP)
(74) Representative: Olgemöller, Luitgard, Dr.
(86) International application number: PCT/JP2003/000359
(87) International publication number: WO 2003/062237

(57) **Abstract**

The objectives of this invention are to provide a luminescent compound capable of emitting white light by itself, and an illuminator and organic EL element that emit white light by the employment of the luminescent compound. The illuminator and the organic EL element are capable of emitting white light at a strong luminance for a long time.

The luminescent compound is represented by the following formula (1) or (2): 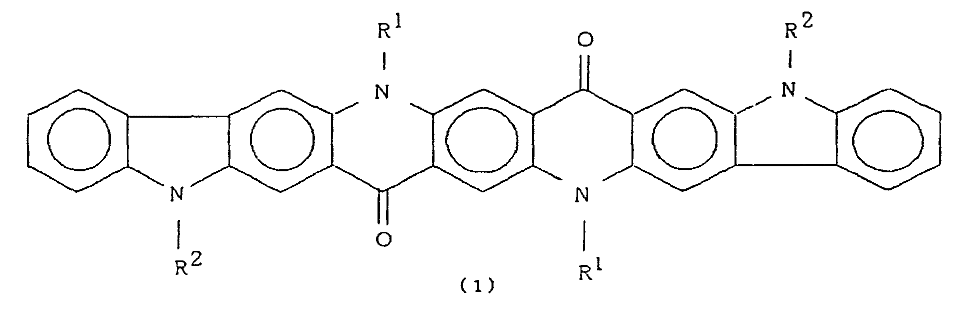 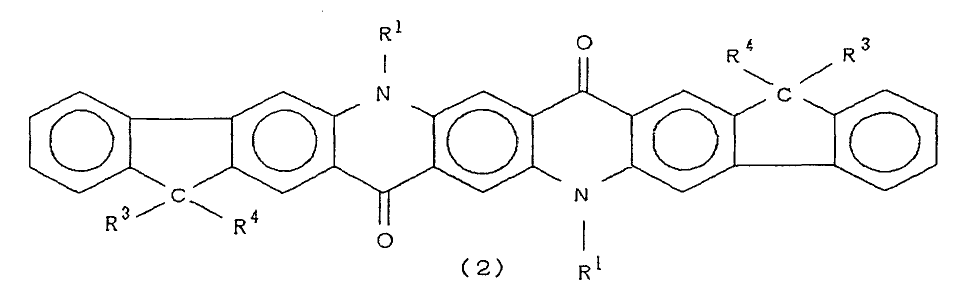 The illuminator and the organic EL element comprise a substrate on which an electrode has been formed and a luminescent layer including the compound, said luminescent layer being placed on the substrate.

## Description

### Technical Field

The present invention relates to a luminescent compound emitting white light, and an illuminator and an organic EL element emitting white light that utilize the luminescent compound. More particularly, this invention relates to a single luminescent compound capable of emitting white light by itself at a high luminance, which luminescent compound has a quinacridone skeleton and therefore is solid and excellent in workability. This invention also provides a long-life illuminator and organic EL element emitting, at a high luminance, white light made from a single emitting material, which illuminator and element utilize at least one of the luminescent compounds of the invention for the emitting material.

### Background Art

Conventional illuminators and organic EL elements emitting white light have, between a pair of electrodes, a light-emitting layer comprising a compound emitting red light (R), a compound emitting green light (G) and a compound emitting blue light (B). The white light was made through the mixing of the three lights from the three compounds.

However, it was difficult to adjust the balance of the colors made by the three different compounds. Besides, the compound emitting red light tends to deteriorate. Therefore even though an illuminator or organic EL element emits white light immediately after the production that has included the step of adjusting the balance of the colors, the illuminator or element emits a colored light with the lapse of time, since the compound emitting red light deteriorates quickly. Another problem of the conventional illuminator or element emitting white light is that the luminance is low. On the other hand, few compounds that emit white light by themselves are known. In addition, conventional such compounds had only a short life, which was undesirable.

An objective of this invention is to provide a luminescent compound emitting white light by itself, which can be used for an illuminator and organic EL element emitting white light. In other words, this invention is to provide a luminescent compound emitting white light that may be used for various white light-emitting bodies including an illuminator or organic EL element. Conventional white light-emitting compounds were made to emit white light by mixing pigments that respectively emit the three primary colors, i.e. red, green and blue, and other plural pigments. In this invention, however, is synthesized a new compound capable of emitting white light by itself, which compound can be adapted to an organic EL element that emits pure white light at a high luminance and has a long life.

Another objective of this invention is to provide, by utilizing the compound emitting white light by itself, an illuminator and an organic EL element emitting white light by the light-emission of the single compound, which illuminator and organic EL element have a long life so that they can emit white light for a long time. Still another objective of this invention is to provide a planar white light-emitting illuminator and a tubular white light-emitting illuminator utilizing the long-life compound emitting white light by itself, which planar and tubular white light-emitting illuminators therefore can emit white light for a long time.

### Summary of the Invention

In order to solve the aforementioned problems, this invention provides a luminescent compound emitting white light that has a structure represented by formula (1): 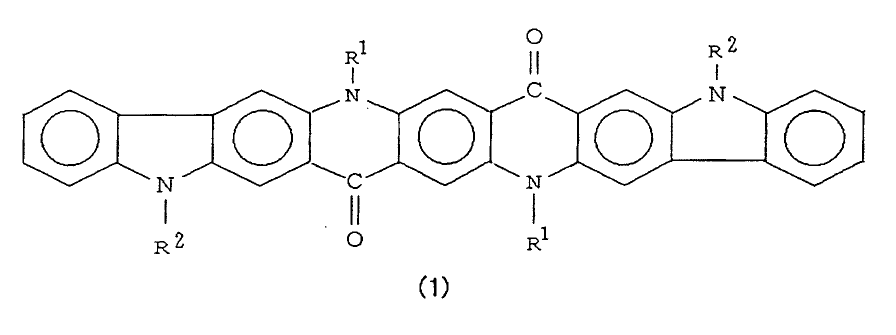 wherein R¹ is hydrogen atom, an alkyl group, or an aryl or alkyl aryl group that may have at least one substituent, wherein two R¹s may be the same or different from each other; R² is hydrogen atom, an alkyl group, or an aryl or alkyl aryl group that may have at least one substituent, wherein two R²s may be the same or different from each other; and R¹ and R² may be the same or different from each other.

Alternatively, this invention provides a luminescent compound emitting white light that has a structure represented by formula (2): 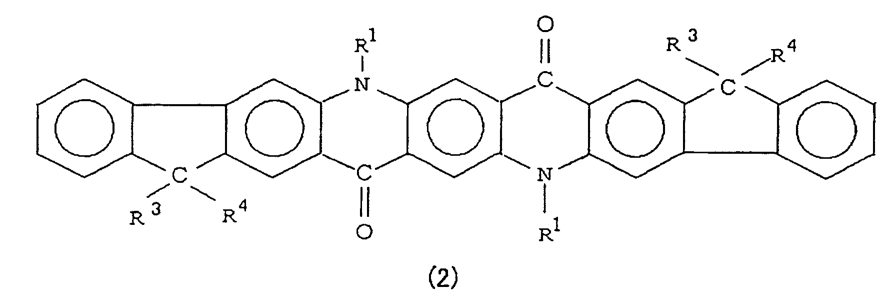 wherein R¹ is hydrogen atom, an alkyl group, or an aryl or alkyl aryl group that may have at least one substituent, wherein two R¹s may be the same or different from each other; each of R³ and R⁴ is hydrogen atom, an alkyl group, or an aryl or alkyl aryl group that may have at least one substituent, wherein R³ and R⁴ may be the same or different from each other; and two R³s may be the same or different and two R⁴s may be the same or different.

As another means to solve the aforementioned problems, this invention provides an illuminator emitting white light comprising a substrate on which an electrode is formed and a light emitting layer which is placed on the substrate, and a light emitting layer comprising a compound emitting white light selected from the group consisting of a first white light-emitting compound represented by formula (1) and a second white light-emitting compound represented by formula (2), wherein each of the compounds emits white light by itself.

In a preferred embodiment of this invention, the illuminator emitting white light is a one-layer type organic EL element comprising the substrate and the light emitting layer which comprises a compound emitting white light selected from the group consisting of the first white light-emitting compound represented by formula (1) and the second white light-emitting compound represented by formula (2), wherein each of the compounds emits white light by itself.

In another preferred embodiment of this invention, the illuminator is a multi-layer type organic EL element comprising the substrate, a hole-transporting layer, the light emitting layer which comprises a luminescent compound emitting white light selected from the group consisting of the first white light-emitting compound represented by formula (1) and the second white light-emitting compound represented by formula (2), and an electron-transporting layer. The light-emitting layer may be a layer prepared by dispersing the compound emitting white light in a high polymer matrix, or by depositing the compound on the substrate. The illuminator may be a planar white light-emitting illuminator or a tubular white light-emitting illuminator.

As still another means to solve the aforementioned problems, this invention provides an organic EL element emitting white light comprising a substrate on which an electrode is formed and a light-emitting layer which is placed on the substrate, and the light-emitting layer which comprises a compound emitting white light selected from the group consisting of a first white light-emitting compound represented by formula (1) and a second white light-emitting compound represented by formula (2), wherein each of the compounds emits white light by itself.

### Brief Description of the Drawings

Figure 1 is an illustration showing a layer structure of the white light-emitting illuminator, which is an example of this invention.
Figure 2 is an illustration showing another layer structure of the white light-emitting illuminator, which is another example of this invention.
Figure 3 is an illustration showing still another layer structure of the white light-emitting illuminator, which is still another example of this invention.
Figure 4 is an illustration showing a further layer structure of the white light-emitting illuminator, which is a further embodiment of this invention.
Figure 5 is an IR chart of the compound prepared by the dehydration reaction of dimethyl-1,4-cyclohexanedion-2,5-dicarboxylate and 3-amino-9-ethylcarbazole in Example 1.
Figure 6 is an IR chart of the intermediate obtained by dehydrogenating the compound prepared by the dehydration reaction in Example 1.
Figure 7 is an NMR chart of the intermediate obtained through the dehydrogenation in Example 1.
Figure 8 is an IR chart of the white light-emitting compound prepared by a ring-closing reaction in Example 1.
Figure 9 is an NMR chart of the white light-emitting compound prepared in Example 1.
Figure 10 is a fluorescent spectrum of the white light-emitting compound prepared in Example 1.
Figure 11 is an IR chart of the compound prepared by the dehydration reaction of dimethyl-1,4-cyclohexanedion-2,5-dicarboxylate and 2-amino-florene in Example 2.
Figure 12 is an IR chart of the intermediate obtained by dehydrogenating the compound prepared by the dehydration reaction in Example 2.
Figure 13 is an IR chart of the white light-emitting compound prepared by a ring-closing reaction in Example 2.
Figure 14 is an IR chart of the compound having the structure represented by formula (16).
Figure 15 is an IR chart of the compound having the structure represented by formula (1b).
Figure 16 is a graph of spectral radiance showing the luminescent properties of an organic EL element including the compound having the structure represented by formula (1b).
Figure 17 is a graph of spectral radiance showing the luminescent properties of another organic EL element including the compound having the structure represented by formula (1b).
Figure 18 is an IR chart of the compound having the structure represented by formula (21).
Figure 19 is an IR chart of the compound having the structure represented by formula (1d).
Figure 20 is a graph of spectral radiance showing the luminescent properties of the organic EL element including the compound having the structure represented by formula (1d).
Figure 21 is a spectrum chart showing a typical emission spectrum and fluorescent spectrum of the luminescent compound emitting white light in accordance with the invention.
Figure 22 is an IR chart of the compound having the structure represented by formula (19).
Figure 23 is an IR chart of the compound having the structure represented by formula (1c).
Figure 24 is an IR chart of the liver brown powder obtained in Example 9.
Figure 25 is an IR chart of the brown powder obtained in Example 9.
Figure 26 is an IR chart of the liver brown powder obtained in Example 10.
Figure 27 is an IR chart of the brown powder obtained in Example 10.
Figure 28 is an IR chart of the liver brown powder obtained in Example 11.
Figure 29 is an IR chart of the brown powder obtained in Example 11.
Figure 30 is an IR chart of the liver brown powder obtained in Example 12.
Figure 31 is an IR chart of the brown powder obtained in Example 12.
Figure 32 is an IR chart of the liver brown powder obtained in Example 13.
Figure 33 is an IR chart of the brown powder obtained in Example 13.
Figure 34 is an IR chart of the liver brown powder obtained in Example 14.
Figure 35 is an IR chart of the brown powder obtained in Example 14.
Figure 36 is an IR chart of the liver brown powder obtained in Example 15.
Figure 37 is an IR chart of the brown powder obtained in Example 15.
Figure 38 is an IR chart of the blue brown powder obtained in Example 16.
Figure 39 is an NMR chart of the blue brown powder obtained in Example 16.
Figure 40 is an IR chart of the compound represented by formula (1e), which is the product synthesized in Example 16.
Figure 41 is an NMR chart of the compound represented by formula (1e), which is the product synthesized in Example 16.
Figure 42 is a graph showing a fluorescent spectrum of the compound emitting white light represented by formula (1e), which is the product obtained in Example 16.

### Detailed Description of the Preferred Embodiments

The compound emitting white light in accordance with the present invention is a novel one represented by formula (1) or (2):

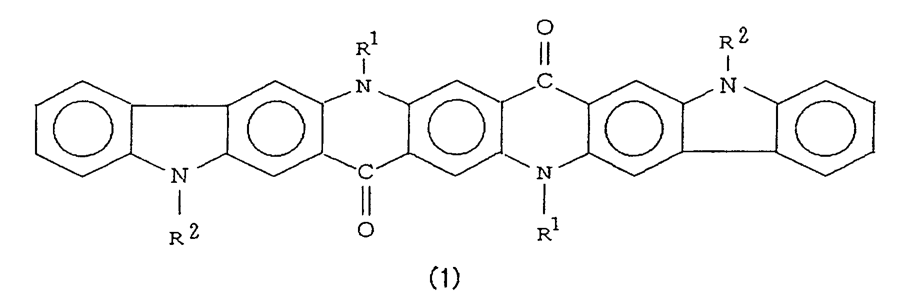

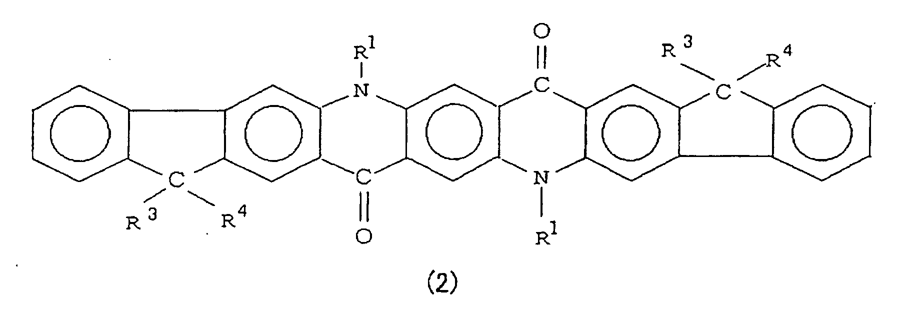

Each of the compounds respectively represented by formulae (1) and (2) has a quinacridone skeleton represented by formula (3):

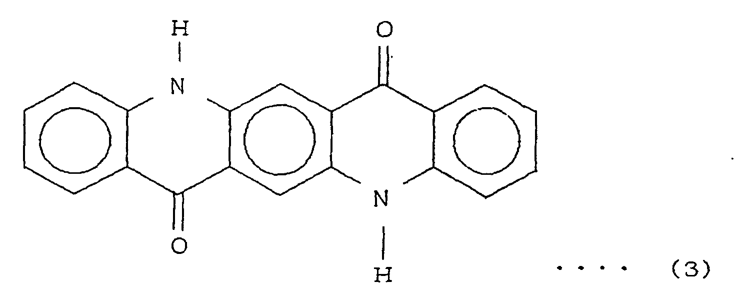

The compound represented by formula (3) is well known as a pigment called quinacridone or pigment violet 19. Quinacridone is synthesized from diethylsuccinyl succinate and aniline, both of which are the main reactants, by, for example, condensation, ring closure and oxidation. Quinacridone per se is excellent in fastness, weatherability and heat resistance. (An excerpt from page 402 of "Shikisai Kogaku Handbook", or "Handbook of Color Technology", edited by Coloring Material Association, Co.)

The white light-emitting compound represented by formula (1) or (2) has a quinacridone skeleton and a carbazole skeleton or carbazole-like skeleton.

Therefore, the white light-emitting compound represented by formula (1) or (2), since it has a skeleton that is solid as a chemical structure, is excellent in fastness, weatherability, light stability and heat resistance. This compound, which has a quinacridone skeleton and a carbazole skeleton or carbazole-like skeleton, is a completely novel compound, which is also excellent in luminescent properties and structural stability.

In formula (1), R¹ denotes hydrogen atom, an alkyl group, or an aryl or alkyl aryl that may have at least one substituent. Suitable for the alkyl group are those having 1-30 carbon atoms, preferably 1-20 carbon atoms, more preferably 1-10 carbon atoms. In particular, a lower alkyl group having 1-5 carbon atoms such as methyl group, ethyl group or propyl group is preferable. Two R¹s may be the same or different from each other. When R¹ is an aryl group, it includes phenyl group, a naphthyl group, an anthryl group and a biphenyl group, and groups derived from these aromatic groups by replacing at least one hydrogen atom thereof with a substituent such as an alkyl group or an alkoxy group. When R¹ is an arylalkyl group, it includes benzyl group, 1-phenylethyl group, 2-phenylethyl group, 1-phenylpropyl group, 2-phenylpropyl group, 3-phenylpropyl group, 5-phenylbutyl group, etc. The phenyl moiety of these groups may further have substituents such as an alkyl group and alkoxyl group. The preferable arylalkyl group is benzyl group.

In formula (1), each of two R²s is hydrogen atom, an alkyl group, or an aryl or arylalkyl group that may have at least one substituent. Two R²s may be the same or different from each other. Suitable for the alkyl group are those having 1-30 carbon atoms, preferably 1-20 carbon atoms, more preferably 1-10 carbon atoms. In particular, a lower alkyl group having 1-5 carbon atoms such as methyl group, ethyl group or propyl group is preferable. Two R²s may be the same or different from each other. When R² is an aryl group, it includes phenyl group, a naphthyl group, an anthryl group and a biphenyl group, and groups derived from these aromatic groups by replacing at least one hydrogen atom thereof with a substituent such as an alkyl group and an al koxy group. An example of the derivative is p-alkoxy phenyl. When R² is an arylalkyl group, it includes benzyl group, 1-phenylethyl group, 2-phenylethyl group, 1-phenylpropyl group, 2-phenylpropyl group, 3-phenylpropyl group, 5-phenylbutyl group, etc. The phenyl moiety of these groups may further have substituents such as an alkyl group and alkoxyl group. The preferable arylalkyl group is benzyl group.

In formula (2), R¹ denote the same atom or group as that explained in respect of formula (1).

In formula (2), each of R³ and R⁴ denotes hydrogen atom, an alkyl group, or an aryl or arylalkyl group that may have at least one substituent. R³ and R⁴ may be the same or different from each other. Suitable for the alkyl group are those having 1-30 carbon atoms, preferably 1-20 carbon atoms, more preferably 1-10 carbon atoms. In particular, a lower alkyl group having 1-5 carbon atoms such as methyl group, ethyl group or propyl group is preferable. One of two R³s may be the same as the other R³, or the former may be different from the latter. Two R⁴s may be the same, or different from the latter. When R³ or R⁴ is an aryl group, it includes phenyl group, a naphthyl group, an anthryl group and a biphenyl group, and groups derived from these aromatic groups by replacing at least one hydrogen atom thereof with a substituent such as an alkyl group and an alkoxy group. An example of the derivative is p-alkoxy phenyl. When R³ or R⁴ is an arylalkyl group, it includes benzyl group, 1-phenylethyl group, 2-phenylethyl group, 1-phenylpropyl group, 2-phenylpropyl group, 3-phenylpropyl group, 5-phenylbutyl group, etc. The phenyl moiety of these groups may further have substituents such as an alkyl group and alkoxyl group. The preferable arylalkyl group is benzyl group.

Substituents R¹, R², R³ and R⁴ provide the white light-emitting compound represented by formula (1) or (2) with improved solubility in a solvent, which, in turn, enhances coating properties when the compound is dissolved in a suitable solvent. Also, these substituents lower the sublimation temperature of the compound, which improves operability in the deposition. Furthermore, the compatibility of the compound with high polymers is improved, which enables the compound to be included in a high polymer film.

The compound emitting white light may be produced in accordance with, for example, the following reaction formula. 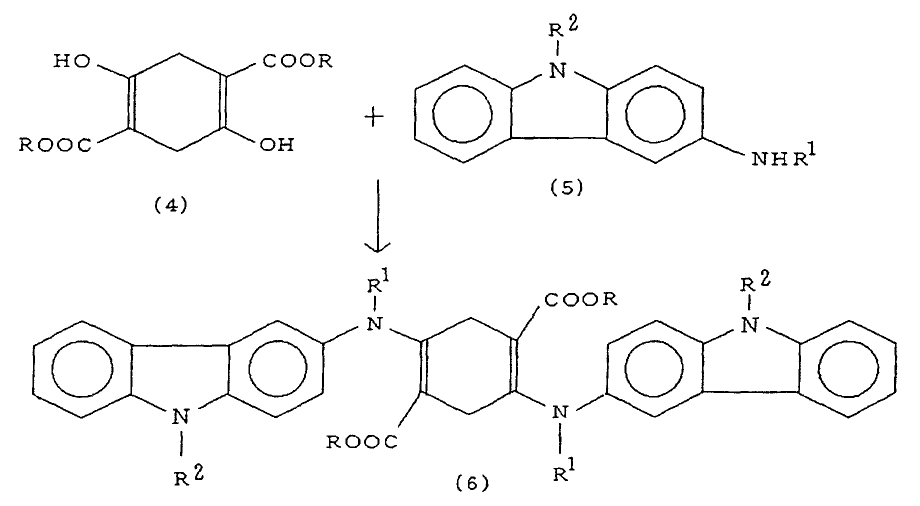 In the reaction formula, R is a lower alkyl group, and R¹ and R² denote the same atoms or groups as those explained above. The reaction proceeds, for example, by heating, in a solvent, dialkyl 2,5-dihydroxy-1,4-cyclohexadiene-1,4-dicarboxylate, which corresponds to a compound represented by chemical formula (4), and such a compound as 3-amino-9-alkylcarbazole, which corresponds to a compound represented by chemical formula (5). In the presence of a catalyst such as acetic acid, the dehydration reaction takes place, which produces the compound having parts bonded by amino groups. This compound is shown by chemical formula (6).

When the dehydrated compound, obtained in the reaction above, is treated with such a dehydrating agent as a concentrated sulfuric acid, the dehydrogenation reaction shown by the following reaction formula takes place.

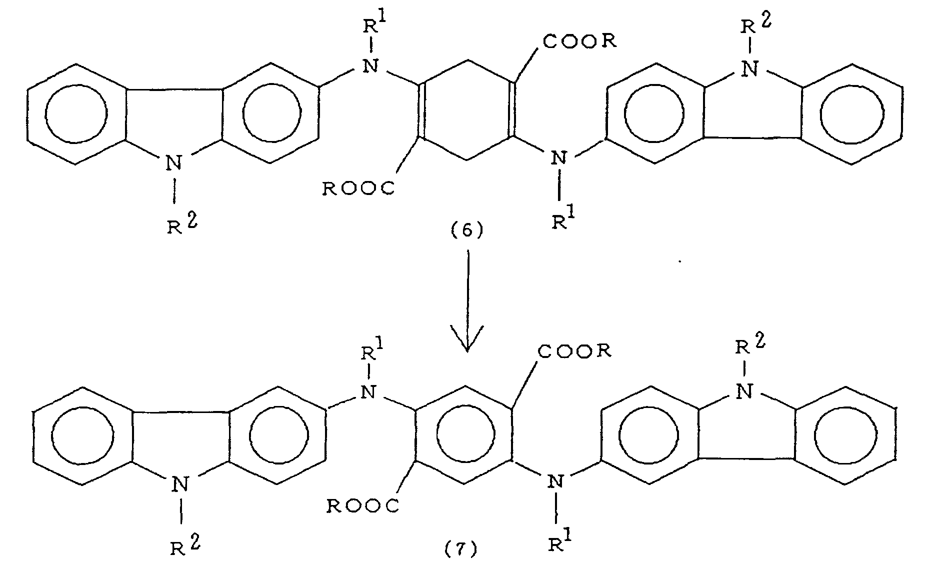

When R¹ is hydrogen atom, the intermediate obtained by the dehydrogenation reaction can be alkylated through the reaction thereof with a halogenated R¹ in, for example, DMF.

Then, the ring-closing reaction in accordance with the following reaction formula is carried out.

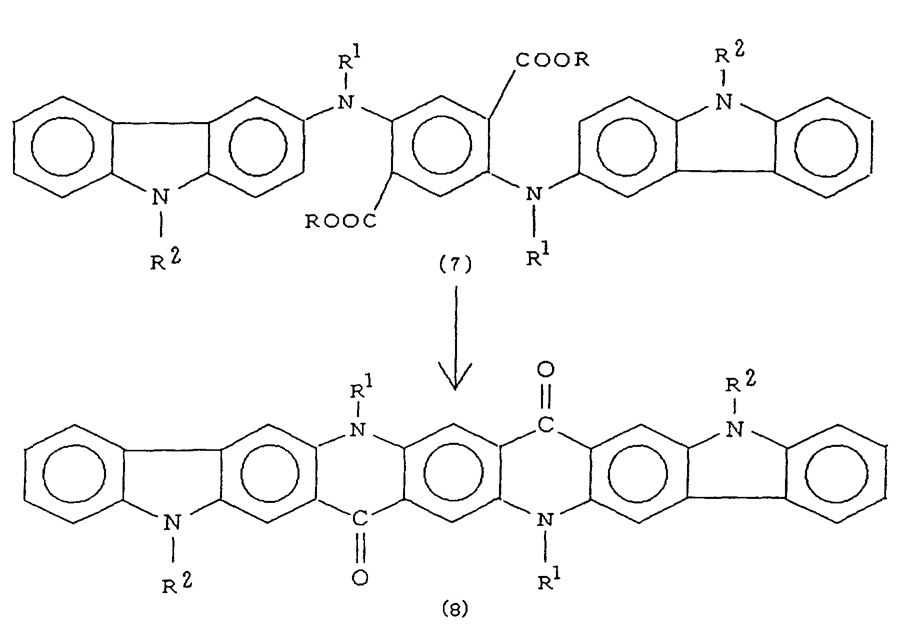

The ring-closing reaction proceeds by heating the intermediate in a solvent, preferably in an inert organic solvent having a high boiling temperature, such as dichlorobenzene, in the presence of an organic acid catalyst such as p-toluenesulfonic acid. After the ring-closing reaction is obtained a white light-emitting compound that includes carbazole skeletons represented by structural formula (8) above.

A compound shown by structural formula (8) is the white light-emitting compound of this invention, the same as that represented by formula (1).

When a compound represented by formula (9) is employed in place of that represented by formula (5), the white light-emitting compound represented by formula (2) is obtained. 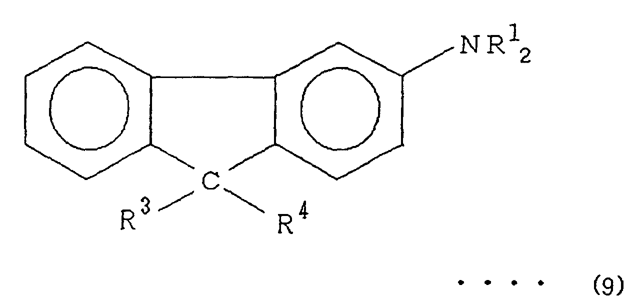 In formula (9), R¹, R³ and R⁴ are the same as those defined hereinbefore.

The reaction between dialkyl 2,5-dihydroxy-1,4-cyclohexadiene-1,4-dicarboxylate, which is a compound represented by chemical formula (4), and a compound represented by formula (9), such as 2-amino florene, proceeds by heating them in a suitable solvent. To subject the reaction product to the dehydrogenation and the ring closure leads to the production of a white light-emitting compound of the invention, represented by formula (2).

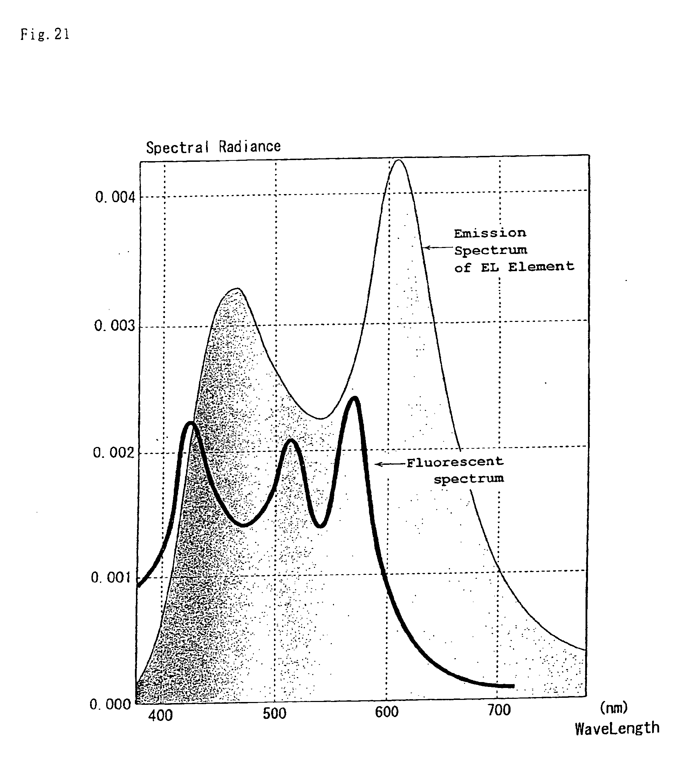

Upon the application of electromagnetic energy, the white light-emitting compound of this invention emits light that has the luminescent spectra respectively corresponding to red, green and blue. The compound emits visible light of which wavelengths range between 400 nm and 700 nm, and typically has such an emission spectrum and fluorescent spectrum as shown in Figure 21. This compound can be utilized for an illuminator or organic EL element capable of emitting white light.

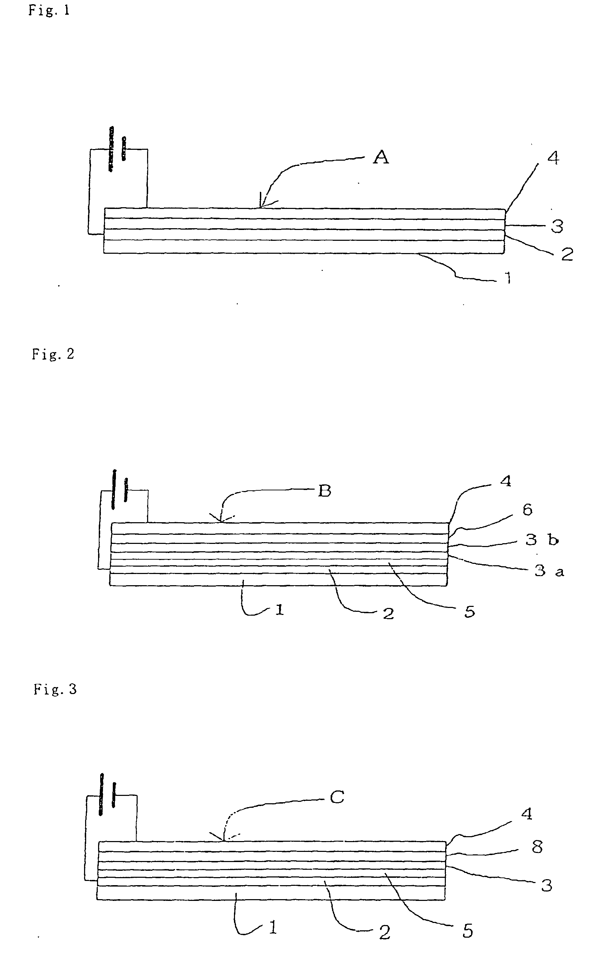

Figure 1 is an illustration showing a layer structure of the white light-emitting illuminator. As shown in Figure 1, a white light-emitting illuminator A comprises a substrate 1 on which a transparent electrode 2 is formed, a light-emitting layer 3 which is placed on the substrate 1, and an electrode layer 4 which is placed on the light-emitting layer 3. When the substrate has a planar shape, the white light-emitting illuminator A will be a planar luminescent illuminator. On the other hand, when the substrate 1 in Figure 1 is shaped into a tube, the white light-emitting illuminator A will be a tubular luminescent illuminator, such as a fluorescent tube. This tubular luminescent illuminator may take the shape of either of a straight tube or a ring tube, such as "CIRCLINE".

For the substrate 1 may be used any known substrate, as long as the transparent electrode 2 can be formed on the surface of the substrate. Examples of the substrate 1 are a glass substrate, a plastic sheet, a ceramic substrate, and a metal substrate of which surface is insulated, for example, through the formation of an insulating layer thereon. When the substrate 1 is opaque, the white light-emitting illuminator is a single-faced one that emits white light from one side of the substrate. On the other hand, when the substrate 1 is transparent, the illuminator is a double-faced one that emits white light from both sides of the substrate.

For the transparent electrode 2, various materials may be employed, as long as their work functions are large, they are transparent, and they can function as a cathode and inject holes into the light-emitting layer 3 when voltage is applied thereto. Specifically, the transparent electrode 2 maybe made of a transparent inorganic conductive material of ITO, In₂O₃, SnO₂, ZnO, CdO, etc. and derivatives thereof, or an electrically conductive high polymer such as polyaniline.

The transparent electrode 2 may be formed on the substrate 1 by chemical vapor phase growth, spray pyrolysis, high-vacuum metal deposition, electron beam deposition, sputtering, ion beam sputtering, ion plating, ion-assisted deposition, and other methods.

When the substrate is made of an opaque material, the electrode formed on the substrate need not be transparent.

The light-emitting layer 3 is a layer that includes a special white light-emitting compound of the invention. The white light-emitting compound will be explained hereinafter. The light-emitting layer 3 may be a high polymer film where the white light-emitting compound is dispersed in a high polymer. The layer may also be a deposited film prepared by depositing the white light-emitting compound on the transparent electrode 2.

Examples of the high polymer for the high polymer film are a polyvinyl carbazole, a poly(3-alkylthiophen), a polyimide including an arylamide, a polyfluorene, a polyphenylene vinylene, a poly-α-methylstyrene, a copolymer of vinylcarbazole and α-methylstyrene. Of them, a polyvinyl carbazole is preferable.

The amount of the white light-emitting compound included in the high polymer film is, typically, 0.01-2 weight%, preferably, 0.05-0.5 weight%.

The thickness of the high polymer film ranges, typically, between 30 nm and 500 nm, preferably between 100 nm and 300 nm. When the thickness is too small, the amount of the emitted light may be insufficient. On the other hand, when the thickness is too large, the voltage required to drive the illuminator or element may be too high, which is not desirable. Besides, the large thickness may reduce the flexibility of the film necessary to shape a planar, tubular, curved or ring article.

The high polymer film may be formed through the application of a solution of the high polymer and the white light-emitting compound of the invention dissolved in a suitable solvent. The application method is one selected from a spin cast method, a coating method, a dipping method, etc.

When the light-emitting layer 3 is a deposited film, the thickness of the film is generally 0.1-100 nm, although a preferable thickness is different depending on the structure of layers and other factors. When the thickness is too large or too small, it might cause the same problems as described above.

For the electrode layer 4 may be employed a material having a small work function. Examples of the material are elementary metals and metallic alloys, such as MgAg, aluminum alloy, metallic calcium, etc. A preferable electrode layer 4 is made of an alloy of aluminum and a small amount of lithium. This electrode may easily be formed on the surface of the light-emitting layer 3, which, in turn, has been formed on the substrate 1, by the technique of metal deposition.

When either of the deposition or the application is employed, a buffer layer should be inserted between the electrode layer and the light-emitting layer.

Materials for the buffer layer are, for example, an alkaline metal compound such as lithium fluoride, an alkaline earth metal compound such as magnesium fluoride, an oxide such as an aluminum oxide, and 4,4'-biscarbazole biphenyl (Cz-TPD). Also, materials for forming the buffer layer between the cathode made of ITO, etc. and the organic layer are, for example, m-MTDATA (4,4',4"-tris(3-methylphenyl- phenylamino)triphenylamine), phthalocyanine, apolyaniline, a polythiophene derivative, and inorganic oxides such as molybdenum oxide, ruthenium oxide, vanadium oxide and lithium fluoride. When the materials are suitably selected, these buffer layers can lower the driving voltage of the organic EL element, which is a white light-emitting illuminator, improve the quantum efficiency of luminescence, and achieve an increase in the luminance of the emitted light.

The white light-emitting illuminator shown in Figure 1, which includes the single luminescent compound capable of emitting white light in the light-emitting layer, emits white light, when an electric current is applied via the transparent electrode 2 and the electrode layer 4.

The following is the explanation for the phenomenon of luminescence. When an electric field is applied to the layer between the transparent electrode 2 and the electrode layer 4, electrons are injected into the layer from the electrode layer 4 and positive holes are injected into the layer from the transparent electrode 2. In the light-emitting layer 3, the electrons are recombined with positive hole, which causes the energy level to return to the valence band from the conduction band. This transition of the energy level is accompanied by emission of the energy differential as light.

The white light-emitting illuminator A shown in Figure 1, when it is shaped into a planar one with a large area, may be used as a large-area wall illuminator fixed on a wall or a large-area ceiling illuminator fixed on a ceiling. This white light-emitting illuminator may be used as a planar light source in place of a point light source, such as a conventional bulb, and a line light source, such as a conventional fluorescent lamp. In particular, this white light-emitting illuminator can suitably be used to light up walls and ceilings in dwelling rooms, offices and passenger trains, or to make them emit light. Moreover, this illuminator may be suitable for the backlight used in the displays of computers, cellular phones and ATMs. Furthermore, this illuminator may be used for various light sources, such as the light source of direct illumination and that of indirect illumination. Also, because it can emit light at night and provide good visibility, it may be used for the light sources of advertisement apparatuses, road traffic sign apparatuses, light-emitting billboards and stop lamps of vehicles. In addition, because the white light-emitting illuminator A includes the white light-emitting compound, which has the special chemical structure, in the light-emitting layer, the illuminator has a long life. Therefore, light sources employing the white light-emitting illuminator A will naturally have a long life.

The white light-emitting illuminator A may also be shaped into a tubular light emitter comprising a tubularly shaped substrate 1, a transparent electrode 2 placed on the inside surface of the substrate 1, a light emitting layer 3 and an electrode layer 4 placed on the transparent electrode 2 in this order. Because this illuminator does not include mercury, it is an ecological light source and may be a substitute for conventional fluorescent lamps.

Another example of the white light-emitting illuminator in accordance with this invention is shown in Figure 2. This figure is an illustration showing the layer structure of an example of the white light-emitting illuminator, which is a multi-layer organic EL element.

As shown in Figure 2, a white light-emitting illuminator B comprises a substrate 1, and a transparent electrode 2, a hole-transporting layer 5, a light-emitting sub layers 3a, 3b and 3c, an electron-transporting layer 6, and an electrode layer 4, the layers being laid on the substrate 1 one layer by one layer in this order.

The substrate 1, the transparent electrode 2 and the electrode layer 4 are the same as those explained for the illuminator A in Figure 1.

The light-emitting layer of the illuminator B comprises light-emitting sub layers 3a and 3b. The light-emitting sub layer 3a is a deposited film including the white light-emitting compound of this invention. The light-emitting sub layer 3b is a DPVBi layer, which functions as a host material.

Examples of the hole-transporting substance included in the hole-transporting layer 5 are a triphenylamine compound such as N,N'-diphenyl-N,N'-di(m-tolyl)-benzidine (TPD) and α -NPD, a hydrazon compound, a stilbene compound, a heterocyclic compound, a π electron star burst positive hole transporting substance, etc.

Examples of the electron-transporting substance included in the electron-transporting layer 6 are an oxadiazole derivative such as 2,5-bis(1-naphthyl)-1,3,4-oxadiazole (BND) and 2-(4-tert-butylphenyl)-5-(4-biphenylyl)-1,3,4-oxadiazole, and 2,5-bis(5'-tert-butyl-2'-benzoxazolyl)-thiophene. Also, a metal complex material such as quinolinol aluminum complex (Alq3), benzoquinolinol beryllium complex (Bebq2) can be used suitably.

The white light-emitting illuminator B shown in Figure 2 employs Alq3 as electron-transporting substance in the electron-transporting layer 6.

The thickness of each layer is the same as that in a known multi-layer organic EL element.

The illuminator B in Figure 2 functions and emits light in the same ways as the illuminator A in Figure 1. Therefore, the illuminator B has the same uses as the illuminator A.

The third example of the white light-emitting illuminator of this invention is shown in Figure 3. This figure is an illustration showing the sectional layer structure of an example of the white light-emitting illuminator, which is a multi-layer organic EL element.

As shown in Figure 3, a white light-emitting illuminator C comprises a substrate 1, and a transparent electrode 2, a hole-transporting layer 5, a light-emitting layer 3, an electron-transporting layer 8, and an electrode layer 4, the electrode and layers being laid on the substrate 1 one layer by one layer in this order.

The illuminator C functions in the same way as the illuminator B.

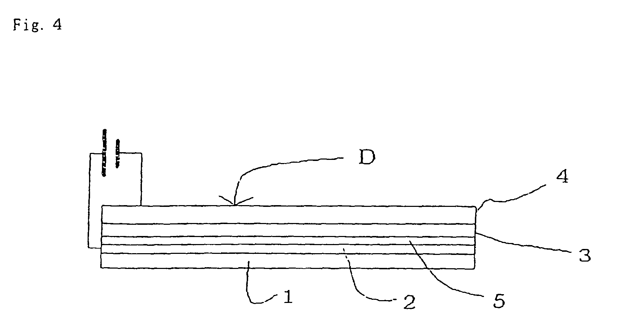

Another example of the white light-emitting illuminator of this invention is shown in Figure 4. A white light-emitting illuminator D comprises a substrate 1, and a transparent electrode 2, a hole-transporting layer 5, a light-emitting layer 3, and an electrode layer 4, the electrode and layers being laid on the substrate 1 one layer by one layer in this order.

An example of the white light-emitting illuminators other than those shown in Figures 1-4, is a two-layer low molecular weight organic luminescent element having a hole-transporting layer that includes a hole-transporting substance and an electron-transporting luminescent layer that includes the white light-emitting compound of the invention laid on the hole-transporting layer, these layers sandwiched between a cathode, which is a transparent electrode formed on the substrate, and an anode, which is an electrode layer. A specific example of this embodiment is a two-layer pigment-injected luminescent element comprising a hole-transporting layer and a luminescent layer that includes a host pigment and the white light-emitting compound of this invention as a guest pigment, wherein the luminescent layer is laid on the hole-transporting layer and these layers are sandwiched between the cathode and the anode. Another example is a two-layer organic luminescent element comprising a hole-transporting layer that includes a hole-transporting substance and an electron-transporting luminescent layer that is prepared through a co-deposition of the white light-emitting compound and an electron-transporting substance, the latter layer being laid on the former, and these two layers being sandwiched between the cathode and the anode. A specific example of the second embodiment is a two-layer pigment-injected luminescent element comprising a hole-transporting layer and an electron-transporting luminescent layer that includes a host pigment and the white light-emitting compound of this invention as a guest pigment, wherein the luminescent layer is laid on the hole-transporting layer and these layers are sandwiched between the cathode and the anode. A further example is a three-layer organic luminescent element comprising a hole-transporting layer, a luminescent layer including the white light-emitting compound of this invention that is laid on the hole-transporting layer, and an electron-transporting layer that is laid on the luminescent layer, these layers being sandwiched between the cathode and the anode.

The electron-transporting luminescent layer, typically, comprises 50-80 weight% of polyvinylcarbazole, which is abbreviated to PVK, 5-40 weight% of an electrode- transporting luminescent agent, and 0.01-20 weight% of the white light-emitting compound of this invention. The electron-transporting luminescent layer of this composition is capable of emitting white light at a high luminance.

Also, it is preferable, if the luminescent layer includes, as a sensitizing agent, rubrene, especially both of rubrene and Alq3.

A white light-emitting illuminator utilizing the compound of this invention may generally be used as an organic EL element which is driven, generally, by direct current, and also by pulses and alternating current.

### Examples

### (Working Example 1)

### <Dehydration Reaction>

In a 1000 ml three-necked flask were placed 25.0 g (1.2 x 10⁻¹ mol) of 3-amino-9-ethylcarbazole, 15.4 g (6.0 x 10⁻² mol) of the compound represented by formula (10), 200 ml of acetic acid and 200 ml of ethyl alcohol. 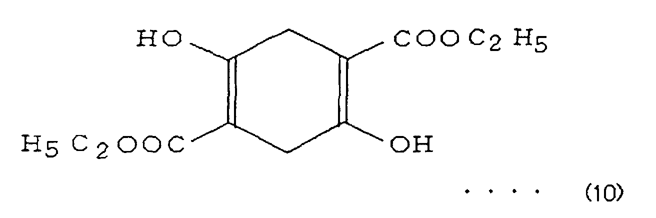 The mixture was placed in a silicone oil bath and heated to 120°C with stirring. The reaction was allowed to continue for 4 hours. After the termination of the reaction, the mixture was cooled to the room temperature.

The obtained reaction mixture was filtered with a glass filter. Solid matter remaining on the filter was washed with methyl alcohol that had been cooled to 5°C and petroleum ether in this order. The washed solid matter was dried in a vacuum. 35.2 g of terracotta powder was obtained.

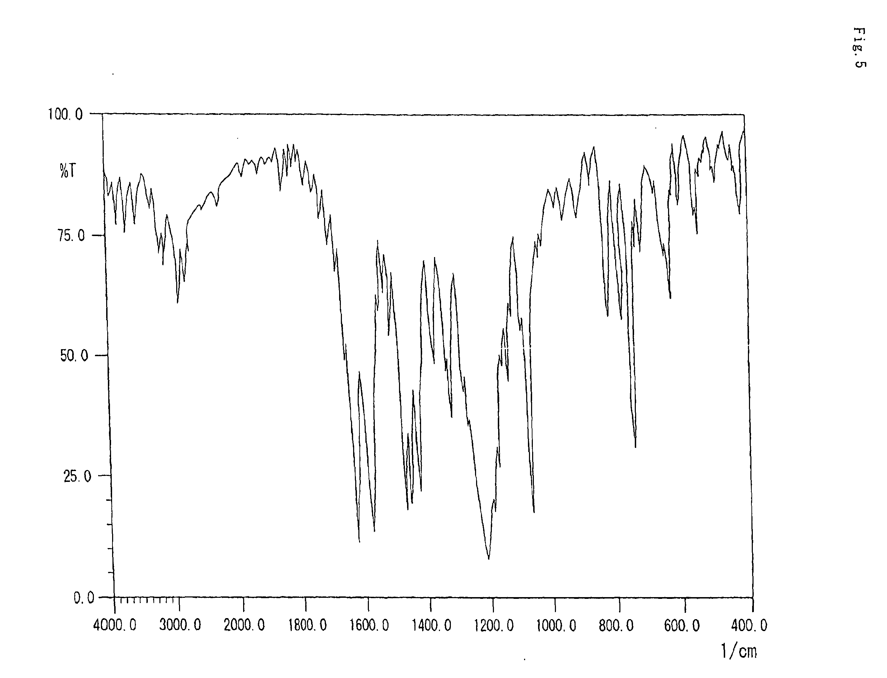

An IR chart of the powder is shown in Figure 5. This terracotta powder was a compound represented by formula (11).

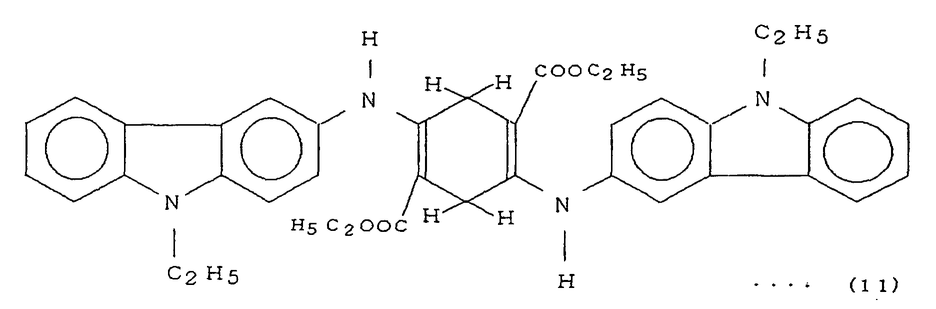

### <Dehydrogenation Reaction>

In a 1000 ml three-necked flask, 25.0 g of the powder produced in the dehydration reaction was placed and 500 ml of o-dichlorobenzene was added thereto. 1.0 g of 95% sulfuric acid was gradually dripped into the flask and stirred for 30 minutes. The mixture was heated to 160°C with stirring in a silicone oil bath and allowed to react for 2 hours. After the termination of the reaction, the reacted mixture was cooled to the room temperature and then introduced into ice water.

The obtained mixture was subjected to extraction with chloroform using a separatory funnel, and the extraction was carried out three times. The extract was washed with water twice and then the moisture included therein was removed with sodium sulfate. The washed and dried was filtered, concentrated and dried up with an evaporator. The obtained solid was washed with acetic acid and petroleum ether in this order, and dried in a vacuum. 13.1 g of red powder was obtained.

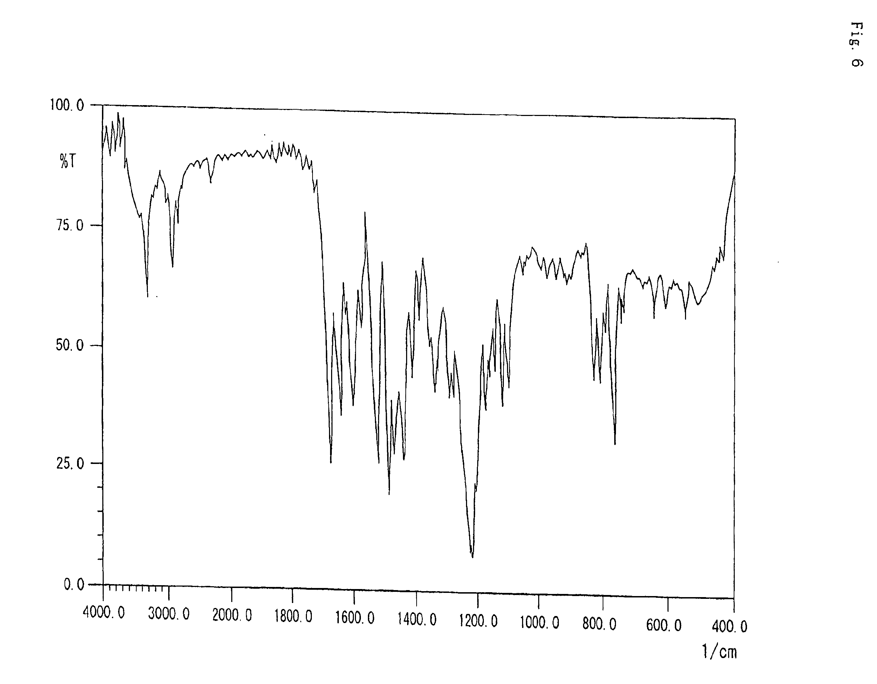

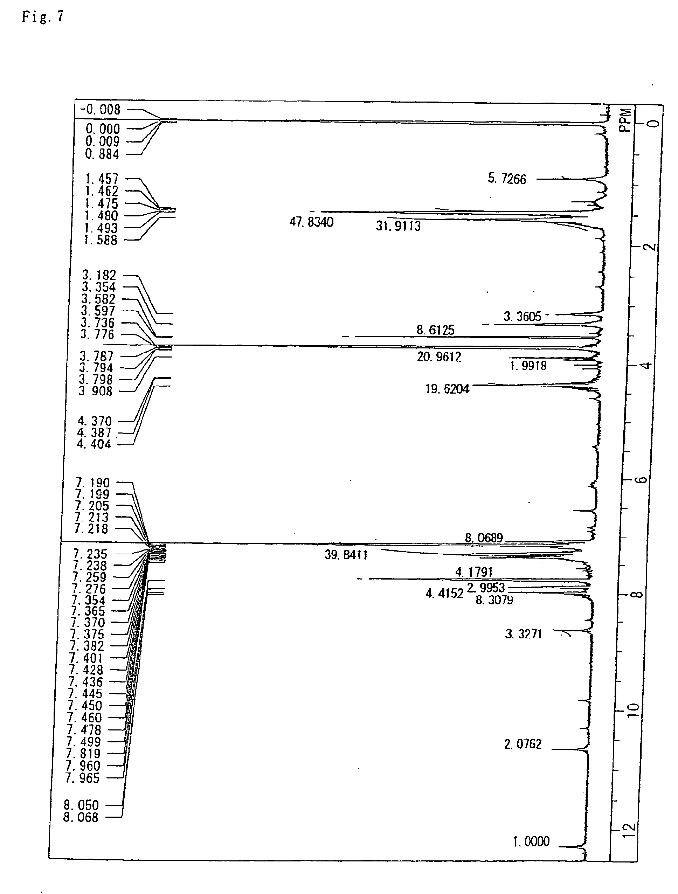

An IR chart of the red powder is shown in Figure 6 and an NMR chart thereof is shown in Figure 7. This red powder had the chemical structure represented by following formula (12).

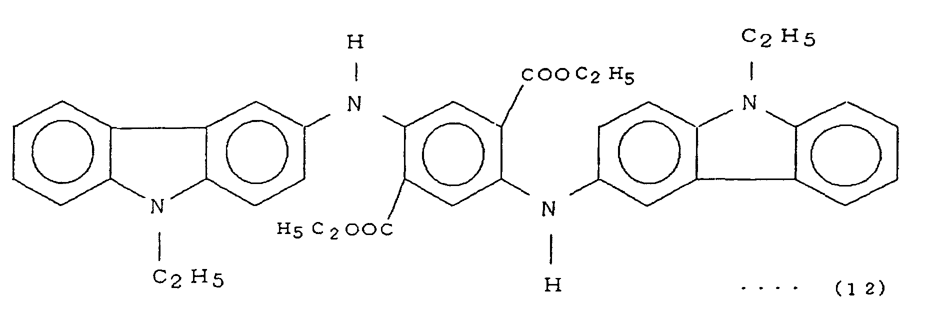

### <Ring-Closing Reaction>

In a 500 ml three-necked flask were placed 10.0 g (1.6 x 10⁻² mol) of the red powder, 13.7 g (7.2 x 10⁻² mol) of hydrated p-toluenesulfonic acid, and 200 ml of o-dichlorobenzene. The mixture was placed in a silicone oil bath and heated to 160°C with stirring. The reaction was allowed to continue for 20 hours. After the termination of the reaction, the reacted mixture was concentrated and dried up with an evaporator. The obtained solid was washed with methyl alcohol that had been cooled to 5°C, acetone, and petroleum ether in this order, and then vacuum dried. 9.4 g of brown powder was obtained. In order to purify the powder, 700 ml of xylene was placed in a 1000 ml Erlenmeyer flask, and 2.0 g of the brown powder and 2.0 g of activated carbon were added to the xylene. The mixture was boiled for 3 minutes with a mantle heater. While the mixture was hot, the liquid was filtered. Then, the obtained filtrate was concentrated and dried up with an evaporator.

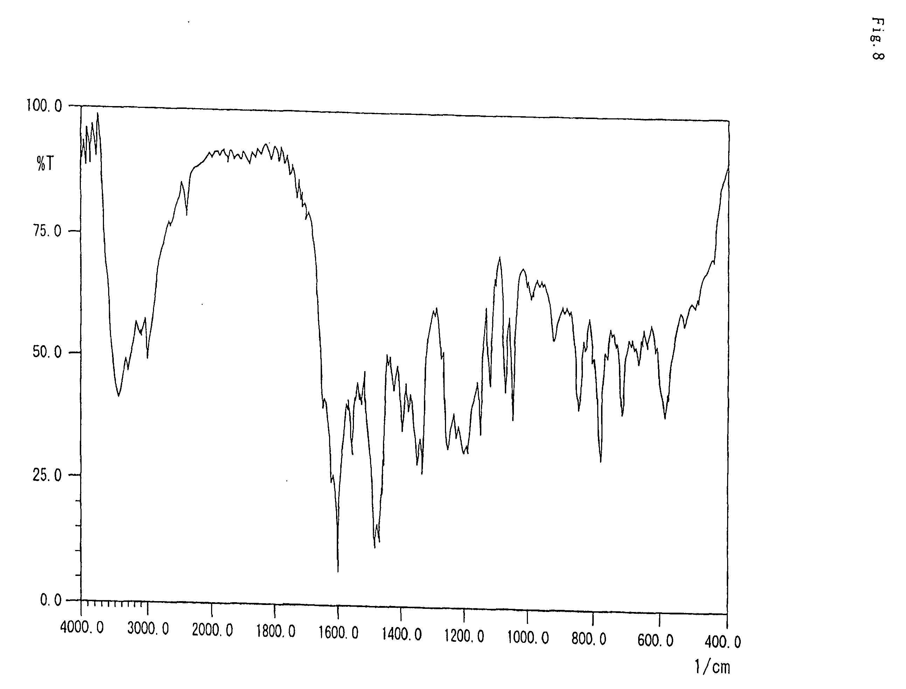

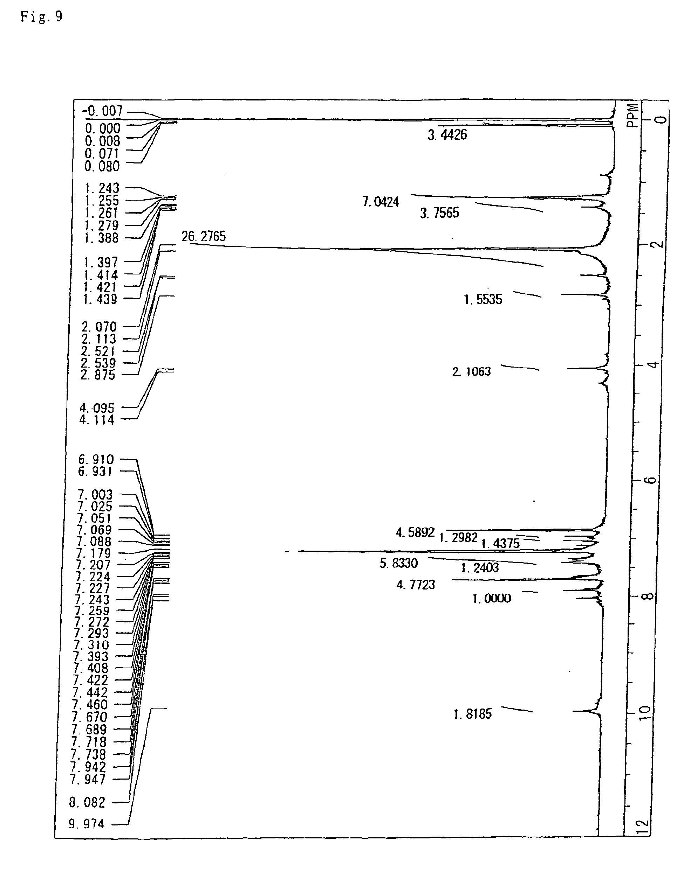

The dry solid was washed with petroleum ether, and dried in a vacuum. Reddish-brown powder was obtained. An IR chart of the reddish-brown powder is shown in Figure 8, and an NMR chart thereof in Figure 9.

This reddish-brown powder was a compound having the chemical structure represented by formula (1a) below.

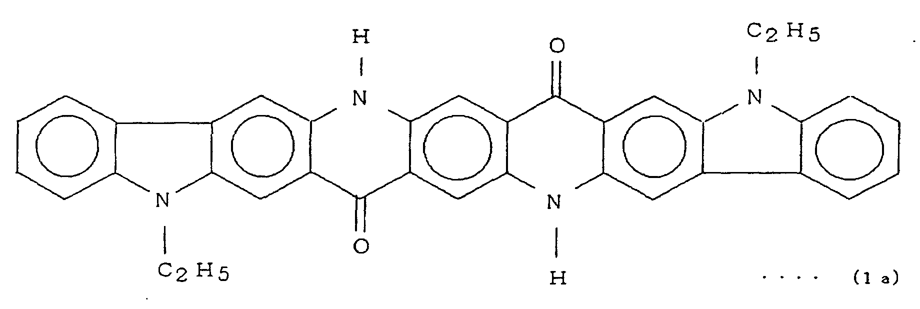

### (Preparation of White Light-emitting Illuminator)

In a 5 ml graduated flask were placed 70 mg of polyvinylcarbazole, 29.7 mg of t-butylphenyl-diphenyl-1,3,4-oxadiazole (PBD) and 0.3 mg of the reddish-brown powder, which is the compound represented by formula (1a). Dichloroethane was added to the flask so that the total volume of the mixture was 5 ml. Thus, a solution including the white light-emitting compound was prepared. This solution was sufficiently homogenized by irradiating it with ultrasound for 20 minutes using a model US-2 ultrasonic cleaner, a product by M.D. Excimer, Inc. An ITO substrate, a product by Sanyo Shinku Industries, Co., Ltd., of which dimensions were 50 x 50 mm and thickness as a transparent electrode was 200 µm, was ultrasonically cleaned in acetone for 10 minutes and then in 2-propanol for 10 minutes. The substrate was blow-dried with nitrogen. Then, the substrate was cleaned by ultraviolet-light irradiation for 30 seconds at a wavelength of 172 nm with a UV irradiator produced by M.D. Excimer, Inc. The solution containing the white light-emitting luminescent compound, which had been prepared, was dropped onto the obtained ITO substrate and a film was formed on the surface of the substrate by spin-coating at 1,500 rpm for 3 seconds with a model 1H-D7 spin coater, a product by Mikasa Co., Ltd., so that the thickness of the film was 100 µm when it was dried. The substrate with the film was dried for 30 minutes in a constant temperature bath at 50°C. The electrode of an aluminum alloy, a product by Kojundo Chemical Laboratory, Co., Ltd., in which the weight ratio of Al to Li was 99:1, was deposited on the substrate under 4 x 10⁻⁶ Torr with a vacuum metallizer (Model VDS-M2-46, produced by DIAVAC Limited). The thickness of the electrode was about 150 nm. Thus a white light-emitting illuminator having a structure shown in Figure 1 was obtained.

The luminance and the chromaticity of the illuminator were measured with a Fast BM-7 measuring apparatus produced by TOPCON Corporation, with the voltage being raised gradually. The results were that, when the voltage was 21 V and the current was 9.69 mA, the luminance was 2, 300 Cd/m², chromaticity X 0.33, and chromaticity Y 0.33.

### <Luminescent Properties>

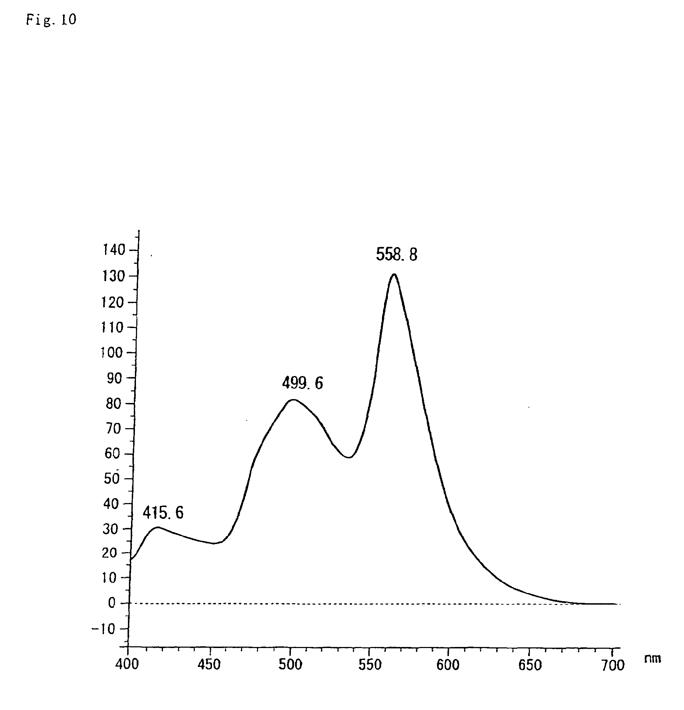

A sample solution was prepared by dissolving the white light-emitting compound represented by formula (1a) in mixed xylene, so that the concentration of the compound was 10 mg/L. This sample solution was loaded in a model F-4500 spectrofluorophotometer, a product by Shimadzu Corporation, and the fluorescence spectrum of the solution was measured under the following conditions. The measured spectrum is shown in Figure 10.

### Conditions of Measurement

Measuring mode: Wavelength scanning
Exciting wavelength: 365 nm
Wavelength at which the emission of fluorescence started: 400 nm
Wavelength at which the emission of fluorescence ended: 700 nm
Scanning speed: 1200 nm/min.
Slit on the side of excitation: 5.0 nm
Slit on the side of fluorescence emission: 5.0 nm
Photomal voltage: 700 V

As understood from Figure 10, the fluorescence of the white light-emitting compound obtained in this example covers a visible light range of which wavelengths are from 400 nm to 700 nm.

### (Working Example 2)

### <Dehydration Reaction>

In a 1000 ml three-necked flask were placed 25.0 g (1.4 x 10⁻¹ mol) of 2-amino-florene represented by formula (13), 17.9 g (7.0 x 10⁻² mol) of the compound represented by formula (10), 200 ml of acetic acid, and 200 ml of ethyl alcohol. 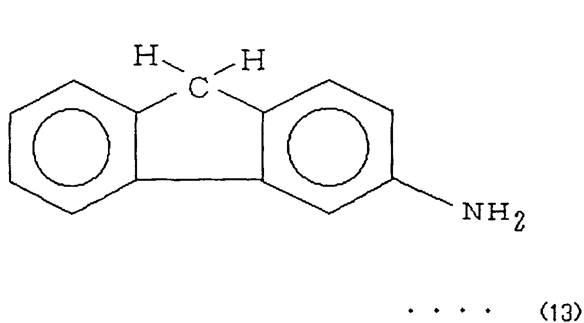 The mixture was placed in a silicone oil bath and heated to 120°C with stirring. The reaction was allowed to continue for 4 hours. After the termination of the reaction, the mixture was cooled to the room temperature.

The obtained reaction mixture was filtered with a glass filter. Solids remaining on the filter were washed with methyl alcohol that had been cooled to 5°C and petroleum ether in this order. The washed solids were dried in a vacuum. 35.4 g of orange powder was obtained.

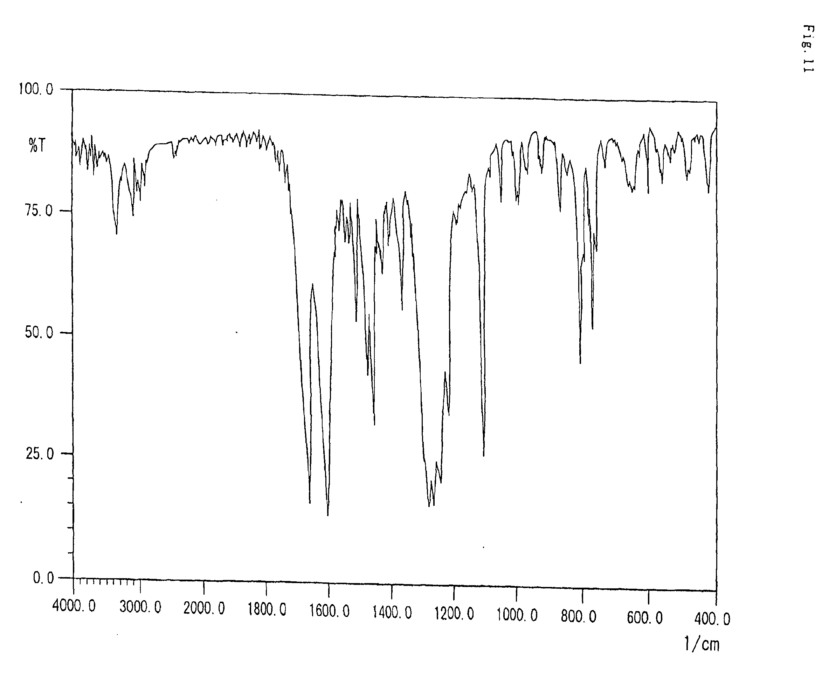

An IR chart of the powder is shown in Figure 11. This orange powder was a compound represented by formula (14).

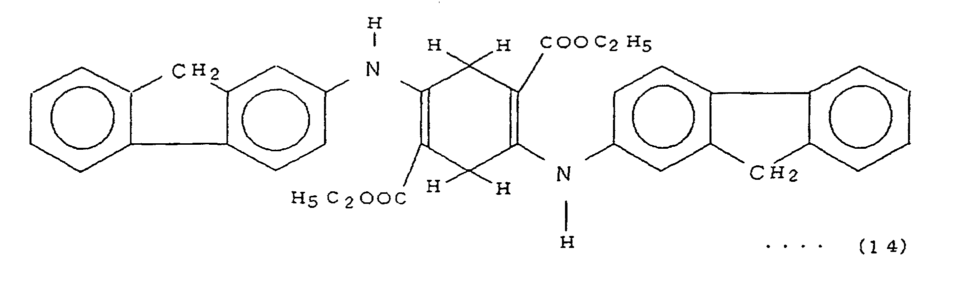

### <Dehydrogenation Reaction>

In a 1000 ml three-necked flask, 25.0 g of the orange powder produced in the dehydration reaction was placed, and 500 ml of o-dichlorobenzene was added thereto. 1.0 g of 95% sulfuric acid was gradually dripped into the flask and stirred for 30 minutes. The mixture was heated to 160°C with stirring in a silicone oil bath and allowed to react for 2 hours. After the termination of the reaction, the reacted mixture was cooled to the room temperature and then poured into ice water.

The obtained mixture was subjected to extraction with chloroform using a separatory funnel, and the extraction was carried out three times. The extract was washed with water twice and then moisture included therein was removed with sodium sulfate. The washed and dried was filtered, concentrated and dried up with an evaporator. The obtained solid was washed with acetic acid and petroleum ether in this order, and dried in a vacuum. 20.5 g of red powder was obtained.

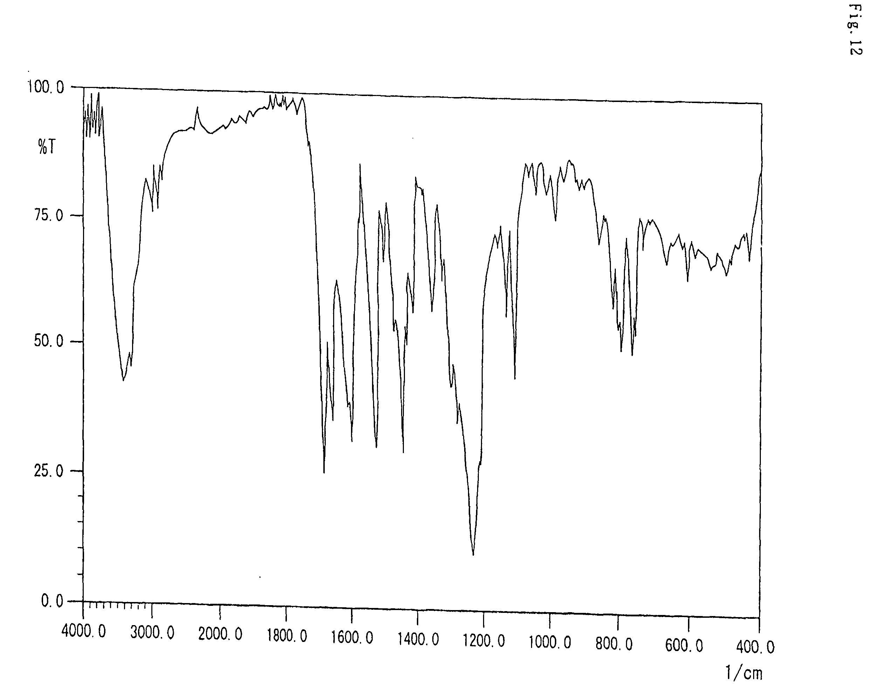

An IR chart of the red powder is shown in Figure 12. This red powder had the chemical structure represented by following formula (15).

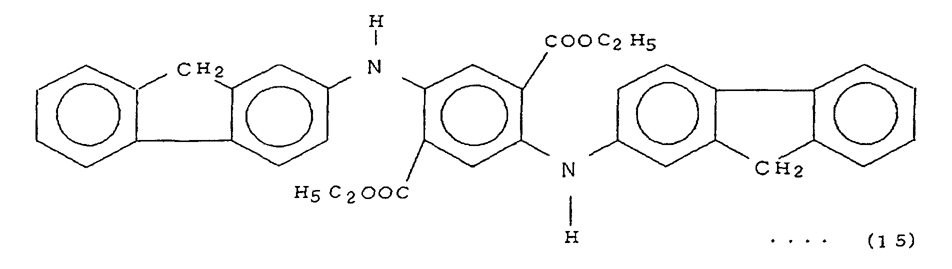

### <Ring-Closing Reaction>

In a 500 ml three-necked flask were placed 10.0 g (1.7 x 10⁻² mol) of the red powder, 15.3 g (8.0 x 10⁻² mol) of hydrated p-toluenesulfonic acid, and 200 ml of o-dichlorobenzene. The mixture was placed in a silicone oil bath and heated to 160°C with stirring. The reaction was allowed to continue for 20 hours. After the termination of the reaction, the reacted mixture was concentrated and dried up with an evaporator. The obtained solid matter was washed with methyl alcohol that had been cooled to 5°C, acetone and petroleum ether in this order, and then vacuum dried. 8.2 g of brown powder was obtained. In order to purify the powder, 700 ml of xylene was placed in a 1000 ml Erlenmeyer flask, and 2.0 g of the brown powder and 2. 0 g of activated carbon were added to the xylene. The mixture was boiled for 3 minutes with a mantle heater. While the mixture was hot, the liquid was filtrated. Then, the obtained filtrate was concentrated and dried up with an evaporator.

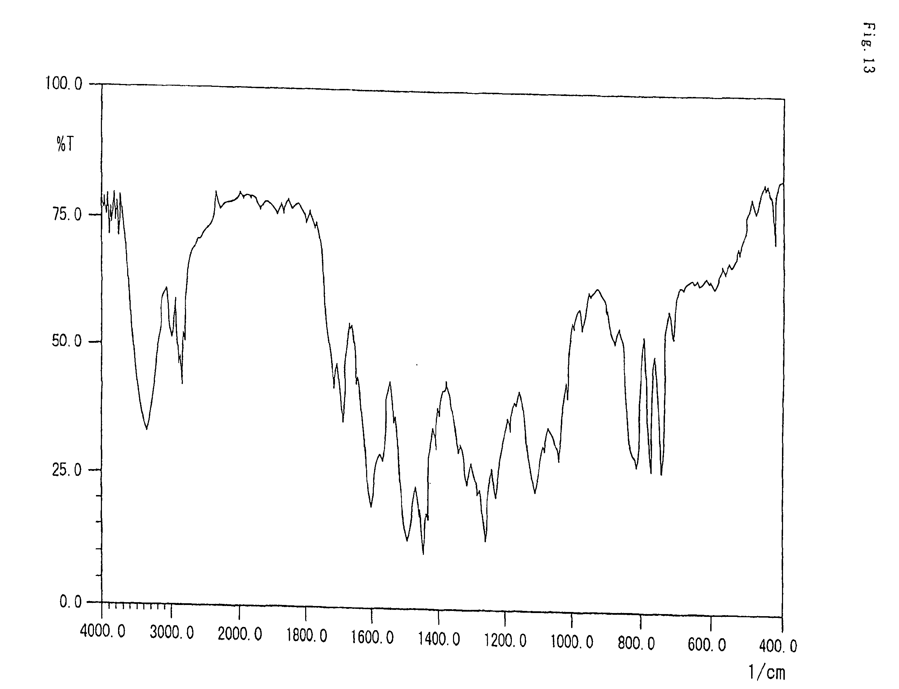

The dry solids were washed with petroleum ether, and dried in a vacuum. Reddish-brown powder was obtained. An IR chart of the reddish-brown powder is shown in Figure 13.

This reddish-brown powder was a compound having the chemical structure represented by formula (2a) below.

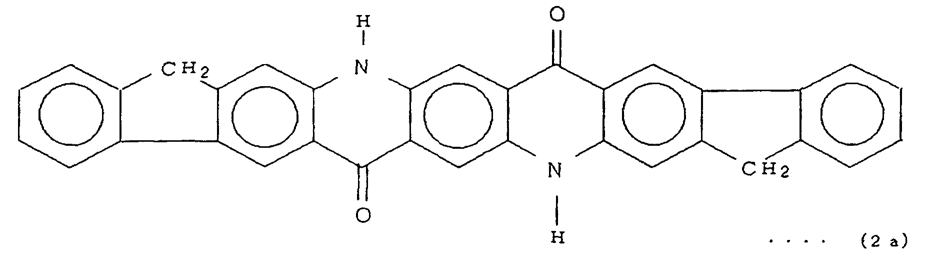

### (Working Example 3)

### <Benzylation Reaction>

In a 500 ml pressure bottle, 10.0 g (1.6 x 10⁻² mol) of the compound represented by formula (12), the same compound as that obtained in Working Example 1 was placed. 17.1 g (7.6 x 10⁻² mol) of benzyl bromide and 200 ml of N, N-dimethylformamide, which will be abbreviated to DMF, were added in this order to the compound. The mixture was heated to 150°C in a silicone oil bath and allowed to react for 20 hours. After the termination of the reaction, the bottle was cooled to the room temperature. Then, the reacted mixture was concentrated with an evaporator. The concentrated mixture was poured into ice water and further neutralized with sodium hydroxide. The neutralized mixture was subjected to extraction with chloroform using a separatory funnel, and the extraction was carried out three times. The extract was washed with water twice and then moisture included therein was removed with sodium sulfate. The washed and dried was filtered, and concentrated and dried up with an evaporator. The obtained solid matter was washed with petroleum ether, and dried in a vacuum. Liver brown powder was obtained.

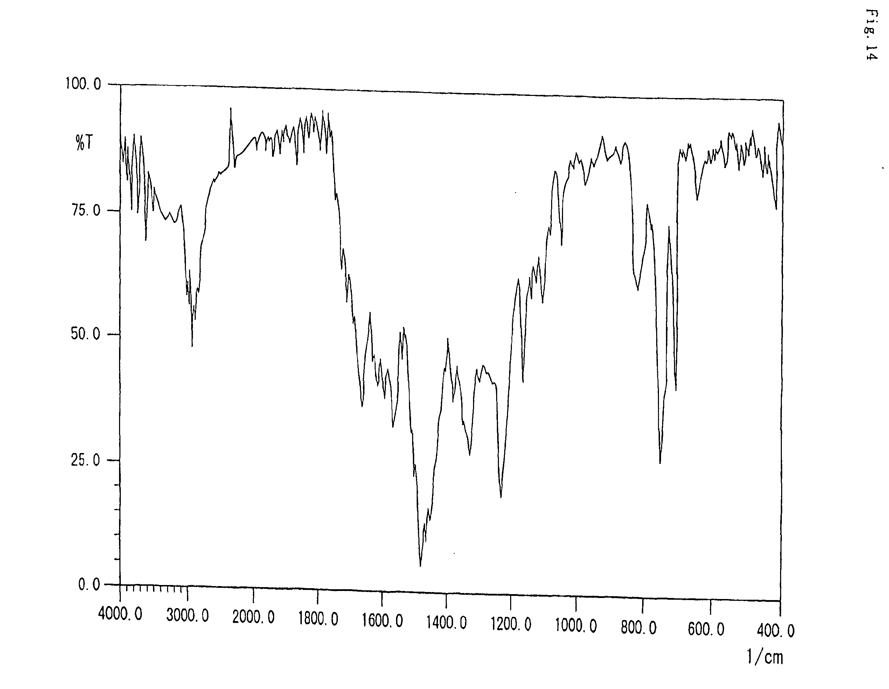

An IR chart of the liver brown powder is shown in Figure 14. This liver brown powder had the chemical structure represented by following formula (16).

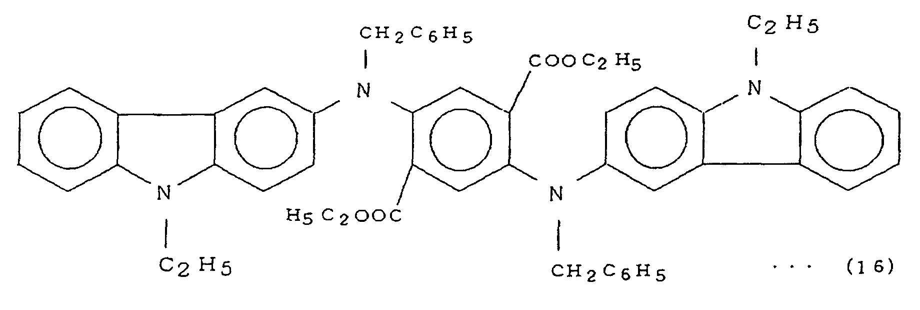

### <Ring-Closing Reaction>

In a 500 ml three-necked flask were placed 5.0 g (6.1 x 10⁻³ mol) of the compound represented by formula (16), 5.2 g (2.7 x 10⁻² mol) of hydrated p-toluenesulfonic acid, and 200 ml of o-dichlorobenzene. The mixture was placed in a silicone oil bath and heated to 160°C with stirring. The reaction was allowed to continue for 20 hours. After the termination of the reaction, the reacted mixture was concentrated and dried up with an evaporator. The obtained solids were washed with methyl alcohol that had been cooled to 5°C, acetone and petroleum ether in this order, and then vacuum dried. Abrown solid matter was obtained.

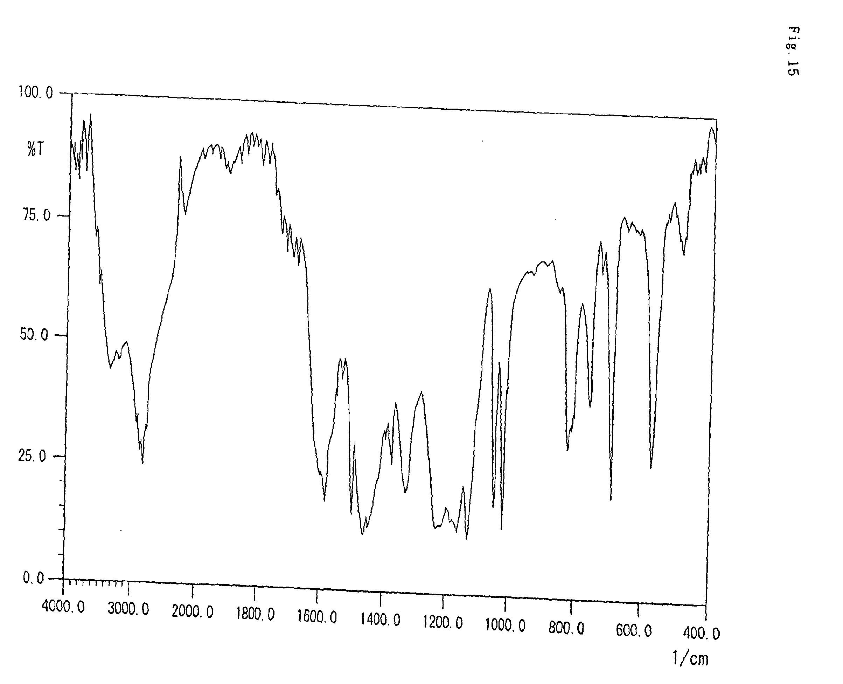

1.0 g of the brown solid matter in 250 ml of xylene was extracted with a Soxhlet abstractor for 24 hours. After the completion of the extraction, the extract was concentrated and dried up with an evaporator. The dried solid was washed with petroleum ether, and dried in a vacuum. Light brown powder was obtained. An IR chart of the light brown powder is shown in Figure 15. This light brown powder was a compound having the chemical structure represented by formula (1b) below.

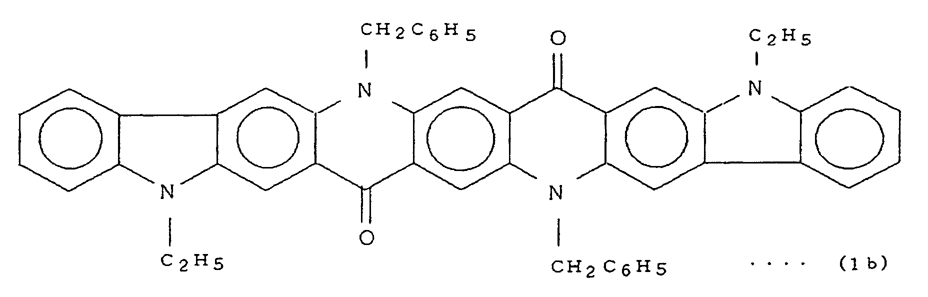

The steps described under the title of (Preparation of White Light-emitting Illuminator) in Working Example 1 were repeated, except the step of preparing the solution including 70 mg of polyvinylcarbazole, 29. 7 mg of t-butylphenyl-diphenyl-1,3,4-oxadiazole (PBD), 0.3 mg of the reddish-brown powder, which is the compound represented by formula (1a), and dichloroethane added so that the total volume of the mixture was 5 ml. In place of the above-mentioned step, the following was carried out: In a 5 ml graduated flask were placed 70 mg of polyvinylcarbazole, 29.7 mg of BND having the structure represented by formula (17) and 0.3 mg of the white light-emitting compound represented by formula (1b). Dichloroethane was added to the placed so that the total volume of the mixture was 5 ml. Thus, a solution including the white light-emitting compound was prepared. With this solution, a white light-emitting illuminator having the structure shown in Figure 1 was prepared in the same way as in Working Example 1. The luminance and the chromaticity of the illuminator were measured with a model SR-3 spectroradiometer produced by TOPCON Corporation.

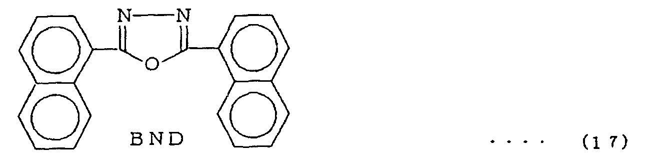

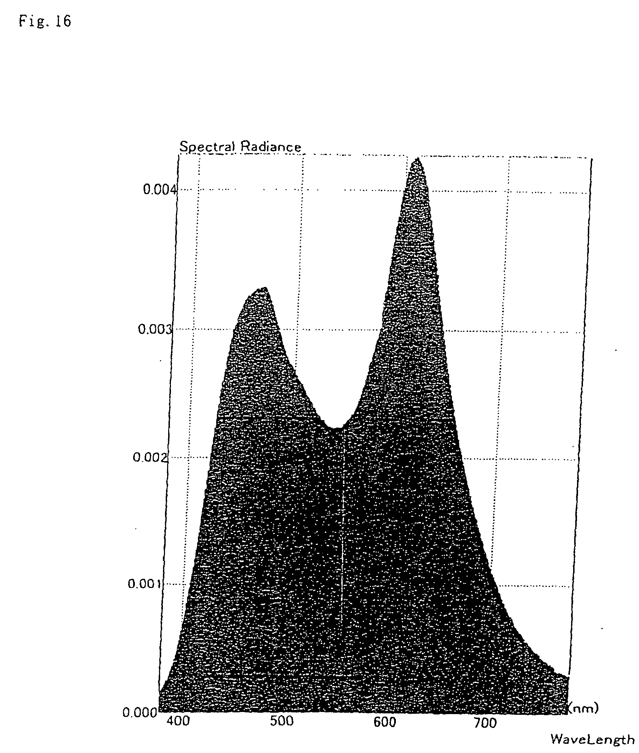

The results were that, when the voltage was 21 V and the current was 36. 8 mA, the luminance was 2, 400 Cd/m², chromaticity X 0. 34, and chromaticity Y 0.35. A graph of the spectral radiance of this white light-emitting compound having been measured with the spectroradiometer is shown in Figure 16. From this graph, it is understood that the color of the emitted light was sufficiently white to the naked eye.

### <Luminescent Properties>

An ITO substrate, a product by Sanyo Shinku Industries, Co., Ltd., of which dimensions were 50 x 50 mm and thickness as a transparent electrode was 200 µm, was ultrasonically cleaned in acetone for 10 minutes and then in 2-propanol for 10 minutes. The substrate was blow-dried with nitrogen. Then, the substrate was cleaned by ultraviolet-light irradiation for 30 seconds at a wavelength of 172 nm with a UV irradiator produced by M.D. Excimer, Inc.

The obtained ITO substrate was set in a model USD-M2-46 vacuum metallizer, a product by DIAVAC Limited. With this apparatus, N,N'-diphenyl-N,N-di(m-tolyl)-benzidine, which will be abbreviated to TPD, was deposited on the substrate, so that the formed layer had a thickness of 63 nm. Then, the white light-emitting compound represented by formula (1b) was deposited on the TPD layer so that the white light-emitting compound layer had a thickness of 0.5 nm. Next, DPVBi having the structure represented by formula (18) was deposited on the compound layer so that the DPVBi layer had a thickness of 35 nm. Furthermore, tris(8-quinolinate) aluminum (Alq3) was deposited on the DPVBi layer so that the Alq3 layer has a thickness of 150 nm. Finally, an electrode was made through the deposition of an aluminum alloy, a product by Kojundo Chemical Laboratory, Co., Ltd., in which the weight ratio of Al to Li was 99:1, on the Alq3 layer. The thickness of the electrode was 150 nm. All of the depositions were carried out under a pressure of 4 x 10⁻⁶ Torr with the vacuum metallizer. Thus a white light-emitting illuminator having the structure shown in Figure 2 was obtained.

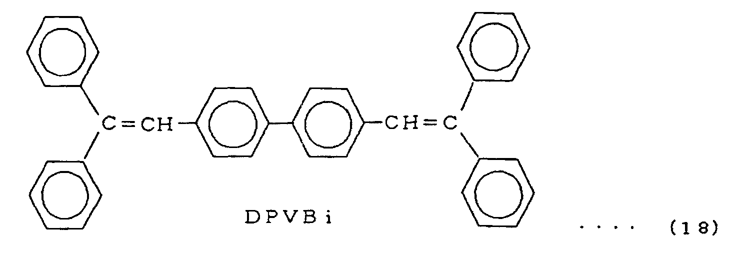

The luminance and the chromaticity of the illuminator were measured with the same method as in Working Example 1.

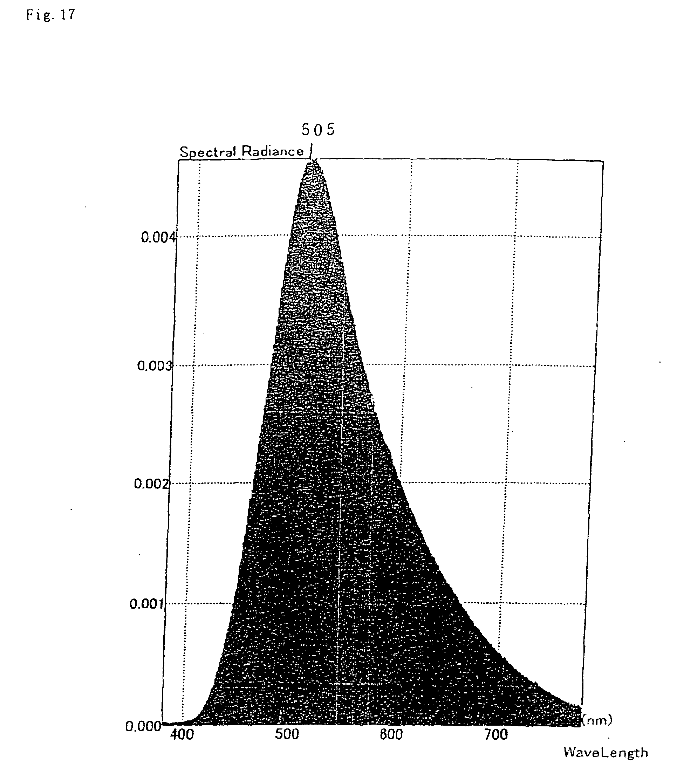

The results were that, when the voltage was 17 V and the current was 21. 8 mA, the luminance was 4, 500 Cd/m², chromaticity X 0. 32, and chromaticity Y 0. 35. Also, a graph of the spectral radiance of this white light-emitting compound that was measured with the spectroradiometer is shown in Figure 17. From this graph, it is understood that the color of the emitted light was sufficiently white to the naked eye.

### (Working Example 4)

A white light-emitting illuminator having the structure shown in Figure 3 was prepared. The preparation steps were the same as those in Working Example 3, except that the thickness of the N,N'-diphenyl-N,N-di(m-tolyl)-benzidine layer was 59 nm, that of the layer of the white light-emitting compound was 0.7 nm, and that of the DPVBi layer was 36 nm. The luminescent properties of the obtained illuminator were measured with the same method as in Working Example 3. The results were that, when the voltage was 20 V and the current was 31.2 mA, the luminance was 3,100 Cd/m², chromaticity X 0.33, and chromaticity Y 0. 34.

### (Working Example 5)

On a transparent polyester film substrate having a thickness of 188 µm, an ITO electrode having a thickness of 200 nm was formed by deposition. On the surface of the ITO electrode, the first layer of α-NPD(di-(naphtyl-phenylamino) diphenyl), the second layer of the white light-emitting compound having the structure represented by formula (1b), the third layer of DPVBi and the fourth layer of Alq3 were laid in this order by deposition. The thickness of the first layer, that of the second layer, that of the third layer and that of the fourth layer were 66 nm, 0.3 nm, 40 nm and 40 nm respectively. On the top of this laminate, an electrode was made through the deposition of an aluminum alloy, a product by Kojundo Chemical Laboratory, Co., Ltd., in which the weight ratio of Al to Li was 99:1, on the fourth layer. The thickness of the electrode was 150 nm. Thus a white light-emitting illuminator having the structure shown in Figure 2 was obtained. Note that light-emitting layer 3 in Figure 2 was made of the second and third layers.

The luminescent properties of the obtained illuminator were measured with the same method as in Working Example 3. As a result, when the voltage was 16 V and the current was 22.6 mA, the luminance was 7,500 Cd/m², chromaticity X 0.30, and chromaticity Y 0.37.

### (Working Example 6)

A white light-emitting illuminator having the structure shown in Figure 2 was prepared. The preparation steps were the same as those in Working Example 5, except that the first layer was made of TPD, the second layer of the white light-emitting compound having the structure represented by formula (1a), the third layer of DPVBi and the fourth layer of Alq3. The thickness of the first layer, that of the second layer, that of the third layer and that of the fourth layer were 64 nm, 0.6 nm, 35 nm and 38 nm respectively. Note that light-emitting layer 3 in Figure 2 was made of the second and third layers.

The luminescent properties of the obtained illuminator were measured with the method as in Working Example 3. As a result, when the voltage was 14 V and the current was 27. 9 mA, the luminance was 5,100 Cd/m², chromaticity X 0.30, and chromaticity Y 0.33.

### (Working Example 7)

### <Methylbenzylation Reaction>

In a 500 ml pressure bottle, 10.0 g (1.6 x 10⁻² mol) of the compound represented by formula (12), the same compound as that obtained in Working Example 1 was placed. 15.8 g (1.1 x 10⁻¹ mol) of α-chloro-p-xylene and 200 ml of N,N-dimethylformamide (DMF) were added in this order to the compound. The mixture was heated to 150°C in a silicone oil bath and allowed to react for 20 hours. After the termination of the reaction, the bottle was cooled to the room temperature. Then, the reacted mixture was concentrated with an evaporator. The concentrated mixture was poured into ice water and further neutralized with sodium hydroxide. The neutralized mixture was subjected to extraction with chloroform using a separatory funnel, and the extraction was carried out three times. The extract was washed with water twice and then moisture included therein was removed with sodium sulfate. The washed and dried was filtered, and concentrated and dried up with an evaporator. The obtained solid was washed with petroleum ether, and dried in a vacuum. Liver brown powder was obtained.

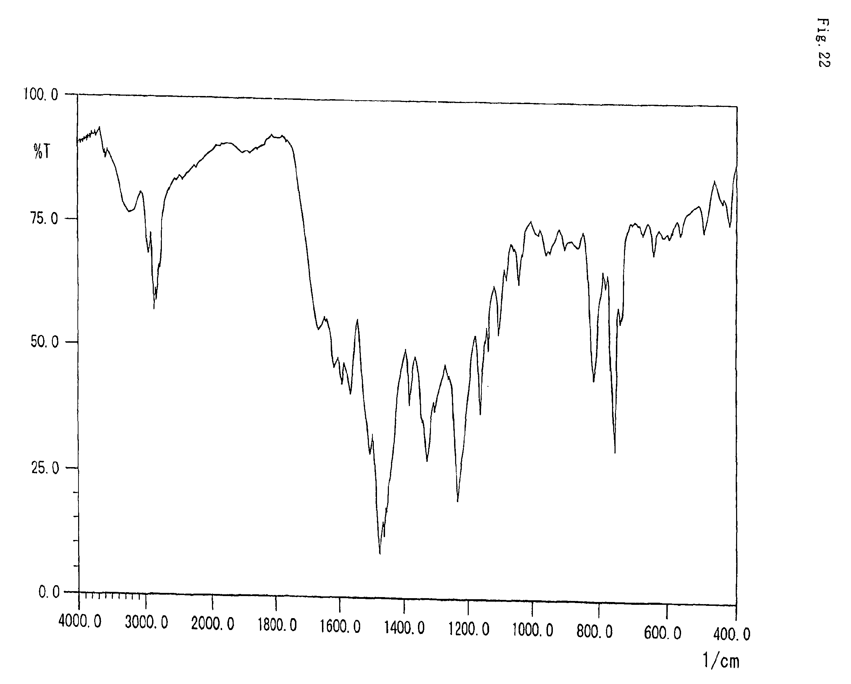

An IR chart of the liver brown powder is shown in Figure 22. This liver brown powder had the chemical structure represented by formula (19).

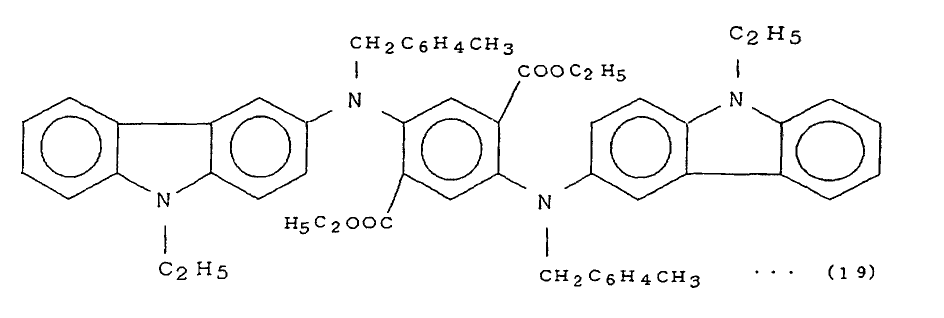

### <Ring Closing Reaction>

In a 500 ml three-necked flask were placed 5.0 g (5.4 x 10⁻³ mol) of the compound represented by formula (19), 8.0 g (4.1 x 10⁻² mol) of p-toluenesulfonic acid monohydrate, and 200 ml of o-dichlorobenzene. The mixture was placed in a silicone oil bath and heated to 160°C with stirring. The reaction was allowed to continue for 20 hours. After the termination of the reaction, the reacted mixture was concentrated and dried up with an evaporator. The obtained solid was washed with methyl alcohol that had been cooled to 5 °C, acetone, and petroleum ether in this order, and then vacuum dried. A brown solid matter was obtained.

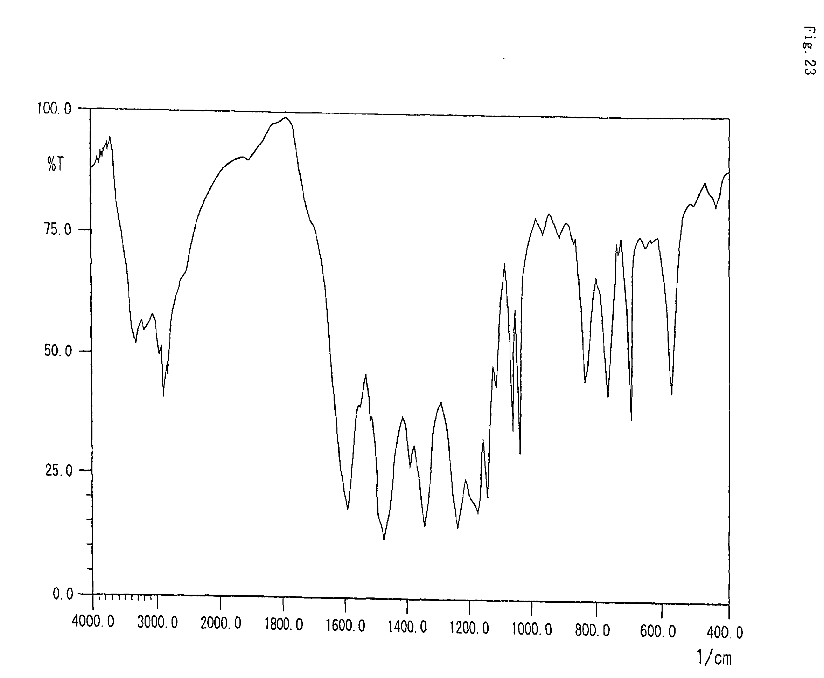

1.0 g of the brown solid matter in 250 ml of xylene was extracted with a Soxhlet abstractor for 24 hours. After the completion of the extraction, the extract was concentrated and dried up with an evaporator. The dried solids were washed with petroleum ether, and dried in a vacuum. Light brown powder was obtained. An IR chart of the light brown powder is shown in Figure 23. This light brown powder was a compound having the chemical structure represented by formula (1c) below.

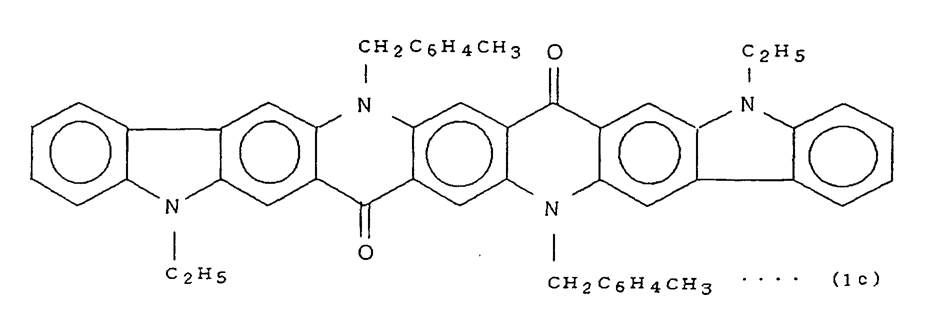

### <White Light-emitting Illuminator>

A white light-emitting illuminator having the structure shown in Figure 3 was prepared. The preparation steps were the same as those in Working Example 3, except that α-NPD, DPVBi doped with 4.4% of the white light-emitting compound having the structure represented by formula (1c), and Alq3 were deposited in this order. The thickness of the α-NPD layer, that of the doped DPVBi layer, and that of the Alq3 layer were 45 nm, 18 nm and 21 nm respectively.

The luminescent properties of the obtained illuminator were measured with the same method as in Working Example 3. The results were that, when the voltage was 18 V and the current was 30.9 mA, the luminance was 10,720 Cd/m², chromaticity X 0.35, and chromaticity Y 0.39.

### <White Light-emitting Illuminator>

A white light-emitting illuminator having the structure shown in Figure 3 was prepared. The preparation steps were the same as those in Working Example 3, except that α-NPD, CBP, which is represented by formula (20) below, doped with 2.9% of the white light-emitting compound having the structure represented by formula (1c), and Alq3 were deposited in this order. The thickness of the α-NPD layer, that of the doped CBP layer, and that of the Alq3 layer were 46 nm, 31 nm and 19 nm respectively.

The luminescent properties of the obtained illuminator were measured with the same method as in Working Example 3. The results were that, when the voltage was 17 V and the current was 36.1 mA, the luminance was 10,120 Cd/m², chromaticity X 0.32, and chromaticity Y 0.35.

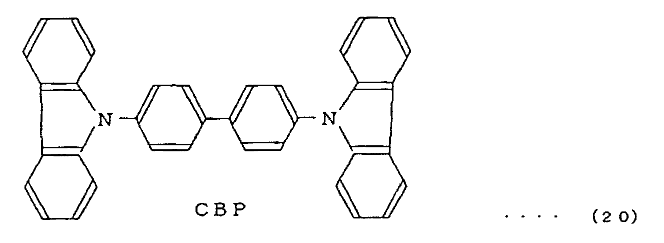

### (Working Example 8)

### <Methylation Reaction>

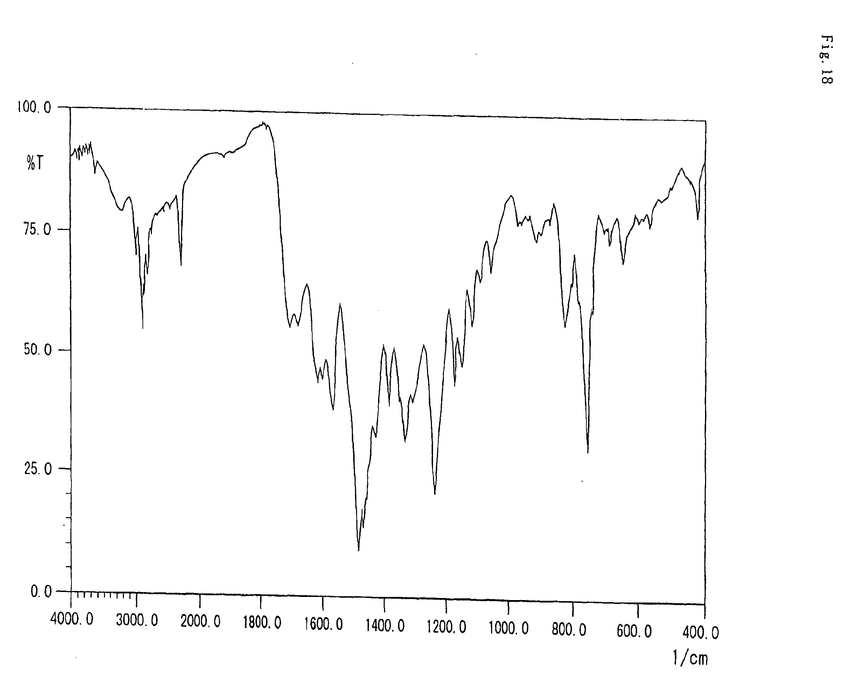

In a 500 ml pressure bottle, 10.0 g (1.6 x 10⁻² mol) of the compound represented by formula (12), the same compound as that obtained in Working Example 1 was placed. 10.8 g (7.6 x 10⁻² mol) of iodomethane and 200 ml of N,N-dimethylformamide (DMF) were added in this order to the compound. The mixture was heated to 150°C in a silicone oil bath and allowed to react for 20 hours. After the completion of the reaction, the bottle was cooled to the room temperature. Then, the reacted mixture was concentrated with an evaporator. The concentrated mixture was poured into ice water and further neutralized with sodium hydroxide. The neutralized mixture was subjected to extraction with chloroform using a separatory funnel, and the extraction was carried out three times. The extract was washed with water twice and then moisture included therein was removed with sodium sulfate. The washed and dried was filtered, and concentrated and dried up with an evaporator. The obtained solid was washed with petroleum ether, and dried in a vacuum. Liver brown powder was obtained. An IR chart of the liver brown powder is shown in Figure 18. This liver brown powder had the chemical structure represented by formula (21).

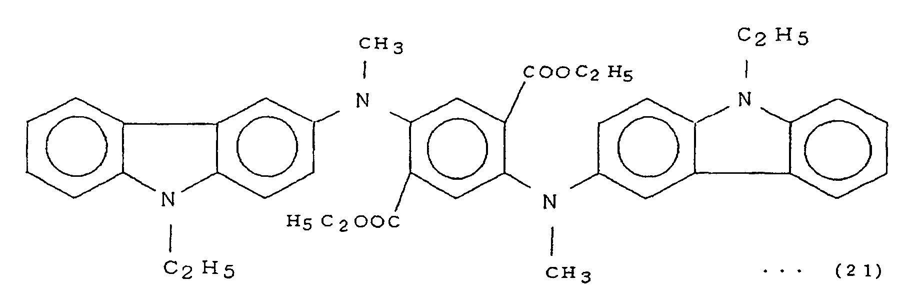

### <Ring Closing Reaction>

In a 500 ml three-necked flask were placed 5.0 g (7.5 x 10⁻³ mol) of the compound represented by formula (21), 8.0 g (4.2 x 10⁻² mol) of p-toluenesulfonic acid monohydrate, and 200 ml of o-dichlorobenzene. The mixture was placed in a silicone oil bath and heated to 160°C with stirring. The reaction was allowed to continue for 20 hours. After the termination of the reaction, the reacted mixture was concentrated and dried up with an evaporator. The obtained solid was washed with methyl alcohol that had been cooled to 5 °C, acetone and petroleum ether in this order, and then vacuum dried. A brown solid matter was obtained.

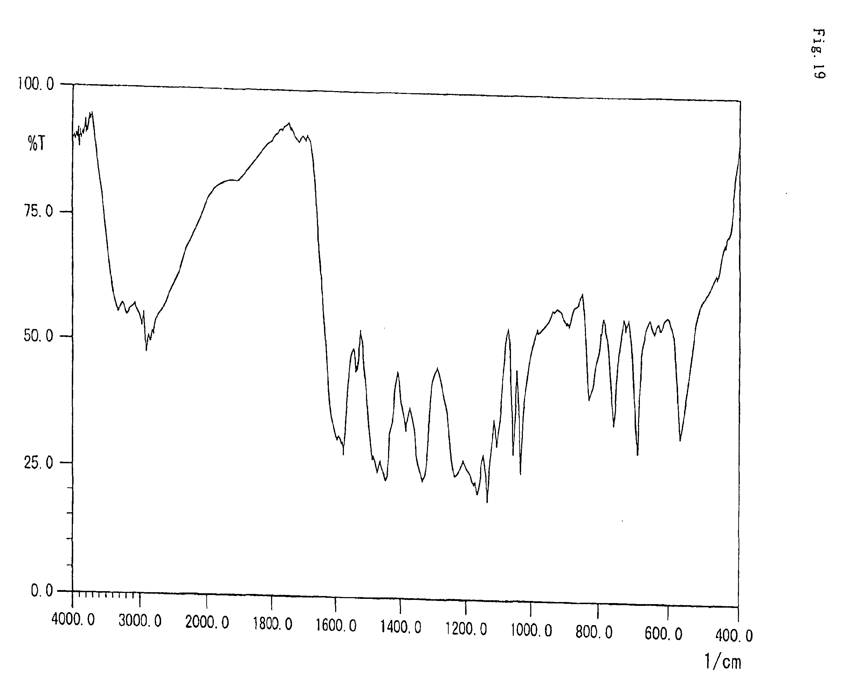

1.0 g of the brown solid matter in 250 ml of xylene was extracted with a Soxhlet abstractor for 24 hours. After the completion of the extraction, the extract was concentrated and dried up with an evaporator. The dried solids were washed with petroleum ether, and dried in a vacuum. Light brown powder was obtained. An IR chart of the light brown powder is shown in Figure 19. This light brown powder was a compound having the chemical structure represented by formula (1d) below.

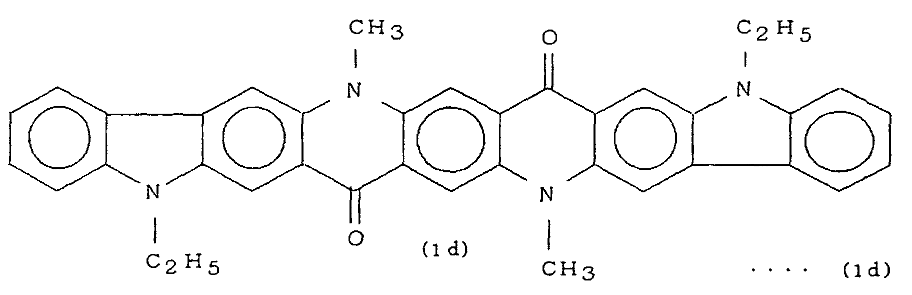

### <White Light-emitting Illuminator>

An EL element was prepared in accordance with the method explained under the subtitle <Luminescent Properties> of Working Example 3. On the surface of the ITO electrode, the first layer of TPD, the second layer of the white light-emitting compound having the structure represented by formula (1d), the third layer of DPVBi and the fourth layer of tris(8-quinolinate)aluminum Alq3 were laid in this order by deposition. The thickness of the first layer, that of the second layer, that of the third layer and that of the fourth layer were 71 nm, 0.6 nm, 40 nm and 41 nm respectively. On the top of this laminate, an electrode was made through the deposition of an aluminum alloy, a product by Kojundo Chemical Laboratory, Co., Ltd., in which the weight ratio of Al to Li was 99:1, on the fourth layer. The thickness of the electrode was 150 nm.

The luminance and chromaticity of the obtained EL element were measured with the same method as in Working Example 3.

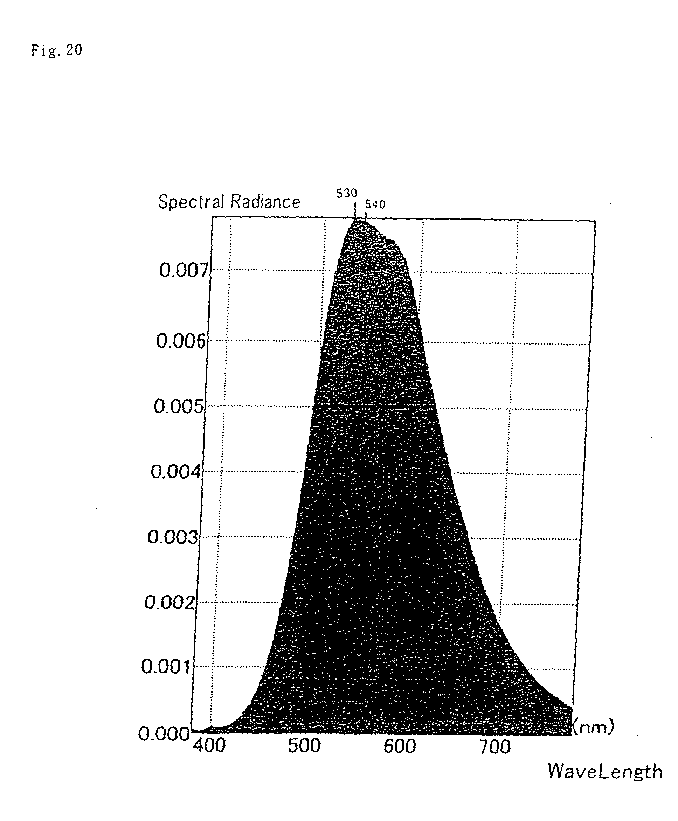

The results were that, when the voltage was 16 V and the current was 17. 5 mA, the luminance was 7, 000 Cd/m², chromaticity X 0.35, and chromaticity Y 0.34. Also, a graph of the spectral radiance of this white light-emitting compound that was measured with the spectroradiometer is shown in Figure 20. From this graph, it is understood that the color of the emitted light was sufficiently white to the naked eye.

### (Working Example 9)

### <Ethylation Reaction>

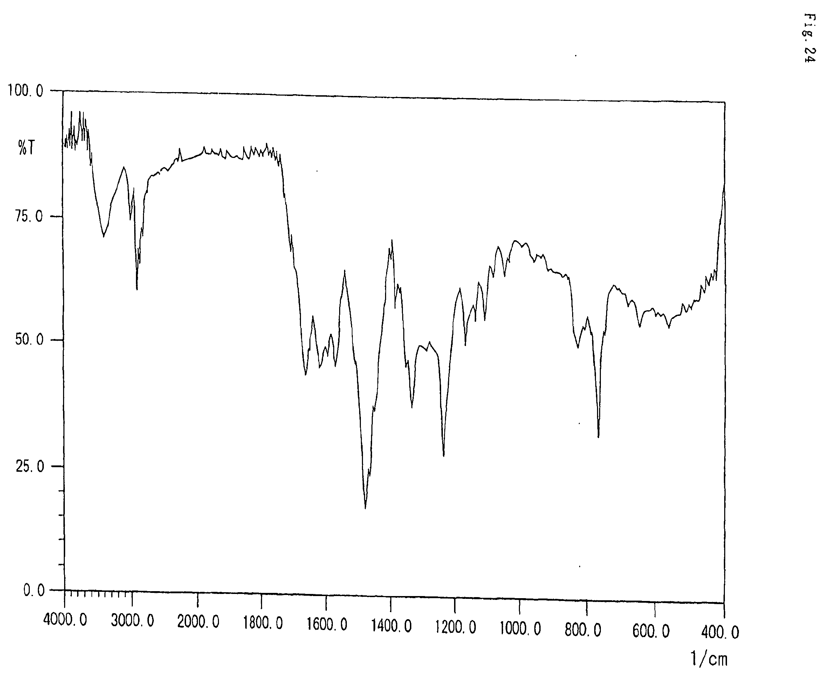

In a 500 ml pressure bottle, 10.0 g (1.6 x 10⁻² mol) of the compound represented by formula (12), the same compound as that obtained in Working Example 1 was placed. 10.8 g (6.9 x 10⁻² mol) of iodoethane (C₂H₅I) and 200 ml of N,N-dimethylformamide (DMF) were added in this order to the compound. The mixture was heated to 150°C in a silicone oil bath and allowed to react for 20 hours. After the completion of the reaction, the bottle was cooled to the room temperature. Then, the reacted mixture was concentrated with an evaporator. The concentrated mixture was poured into ice water and further neutralized with sodium hydroxide. The neutralized mixture was subjected to extraction with chloroform using a separatory funnel, and the extraction was carried out three times. The extract was washed with water twice and then moisture included therein was removed with sodium sulfate. The washed and dried was filtered, and concentrated and dried up with an evaporator. The obtained solid was washed with petroleum ether, and dried in a vacuum. Liver brown powder was obtained. An IR chart of the liver brown powder is shown in Figure 24.

### <Ring Closing Reaction>

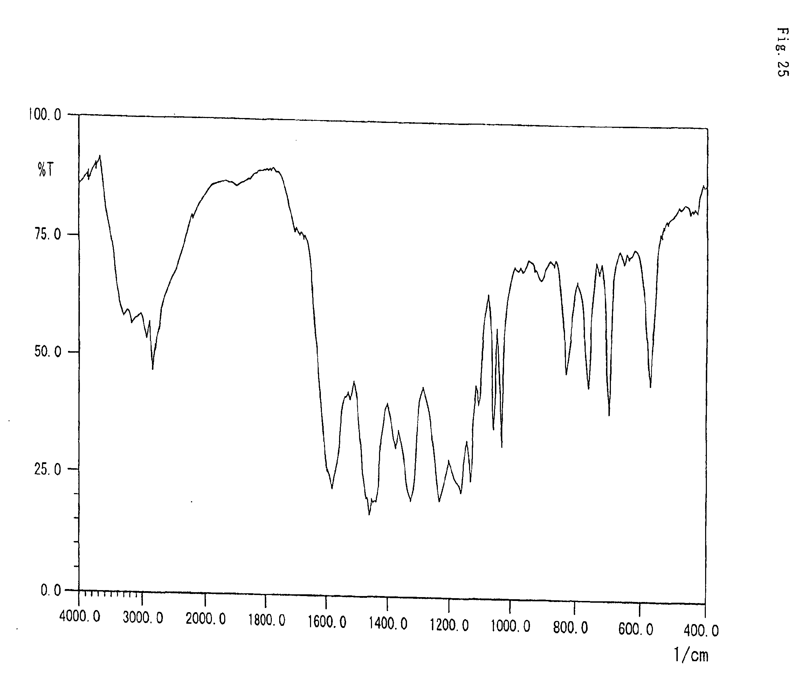

In a 500 ml three-necked flask were placed 5.0 g (8.3 x 10⁻³ mol) of the product obtained in the preceding ethylation reaction, 8.0 g (4.2 x 10⁻² mol) of p-toluenesulfonic acid monohydrate, and 200 ml of o-dichlorobenzene. The mixture was placed in a silicone oil bath and heated to 160°C with stirring. The reaction was allowed to continue for 20 hours. After the termination of the reaction, the reacted mixture was concentrated and dried up with an evaporator. The obtained solid was washed with methyl alcohol that had been cooled to 5 °C, acetone, and petroleum ether in this order, and then vacuum dried. Brown powder was obtained. An IR chart of this brown powder is shown in Figure 25.

### <Purification>

1.0 g of the brown powder obtained in the ring-closing reaction in 250 ml of xylene, which was heated to 185°C using a silicone oil bath, was extracted for 24 hours with a Soxhlet abstractor. After the completion of the extraction, the extract was concentrated and dried up with an evaporator. The dried solid was washed with petroleum ether, and dried in a vacuum. Light brown powder was obtained.

### (Working Example 10)

### <Butylation Reaction>

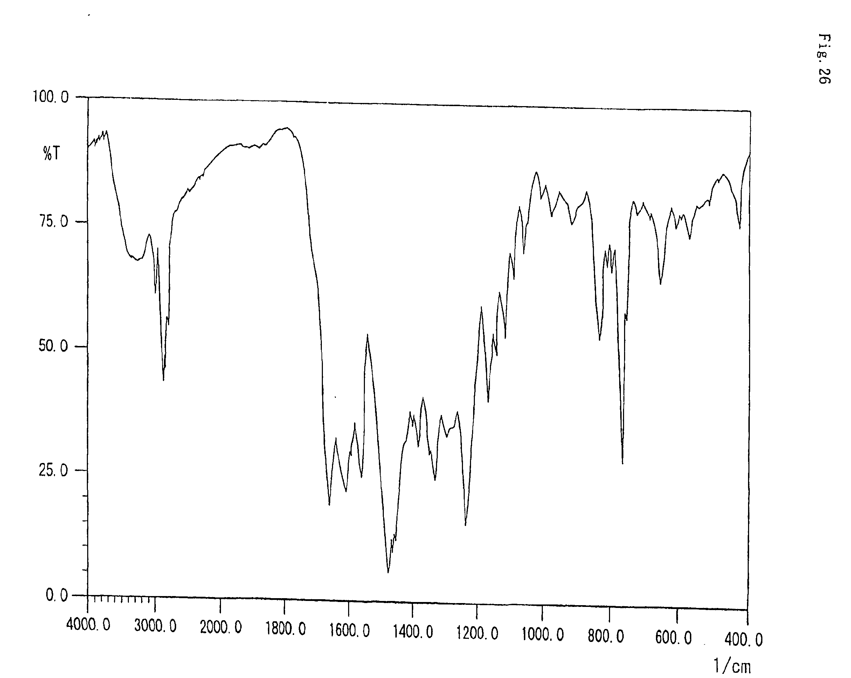

In a 500 ml pressure bottle, 10.0 g (1.6 x 10⁻² mol) of the compound that had the structure represented by formula (12), the same compound as that obtained in Working Example 1 was placed. 10.8 g (7.6 x 10⁻² mol) of n-butyl iodide (C₄H₉I) and 200 ml of N, N-dimethylformamide (DMF) were added in this order to the compound. The mixture was heated to 150°C in a silicone oil bath and allowed to react for 20 hours. After the completion of the reaction, the bottle was cooled to the room temperature. Then, the reacted mixture was concentrated with an evaporator. The concentrated mixture was poured into ice water and further neutralized with sodium hydroxide. The neutralized mixture was subjected to extraction with chloroform using a separatory funnel, and the extraction was carried out three times. The extract was washed with water twice and then moisture included therein was removed with sodium sulfate. The washed and dried was filtered, and concentrated and dried up with an evaporator. The obtained solid was washed with petroleum ether, and dried in a vacuum. Liver brown powder was obtained. An IR chart of the liver brown powder is shown in Figure 26.

### <Ring Closing Reaction>

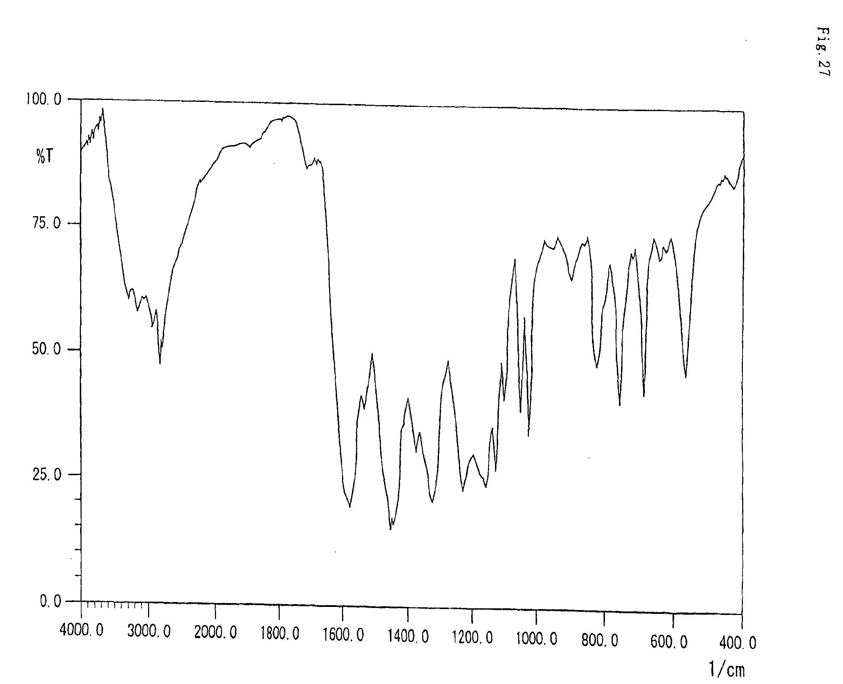

In a 500 ml three-necked flask were placed 5.0 g (7.5 x 10⁻³ mol) of the product obtained in the preceding butylation reaction, 7.2 g (3.7 x 10⁻² mol) of p-toluenesulfonic acid monohydrate, and 200 ml of o-dichlorobenzene. The mixture was placed in a silicone oil bath and heated to 160°C with stirring. The reaction was allowed to continue for 20 hours. After the termination of the reaction, the reacted mixture was concentrated and dried up with an evaporator. The obtained solid was washed with methyl alcohol that had been cooled to 5°C, acetone and petroleum ether in this order, and then vacuum dried. Brown powder was obtained. An IR chart of this brown powder is shown in Figure 27.

### <Purification>

1.0 g of the brown powder obtained in the ring closing reaction in 250 ml of xylene, which was heated to 185°C using a silicone oil bath, was extracted for 24 hours with a Soxhlet abstractor. After the completion of the extraction, the extract was concentrated and dried up with an evaporator. The dried solid was washed with petroleum ether, and dried in a vacuum. Light brown powder was obtained.

### (Working Example 11)

### <Hexylation Reaction>

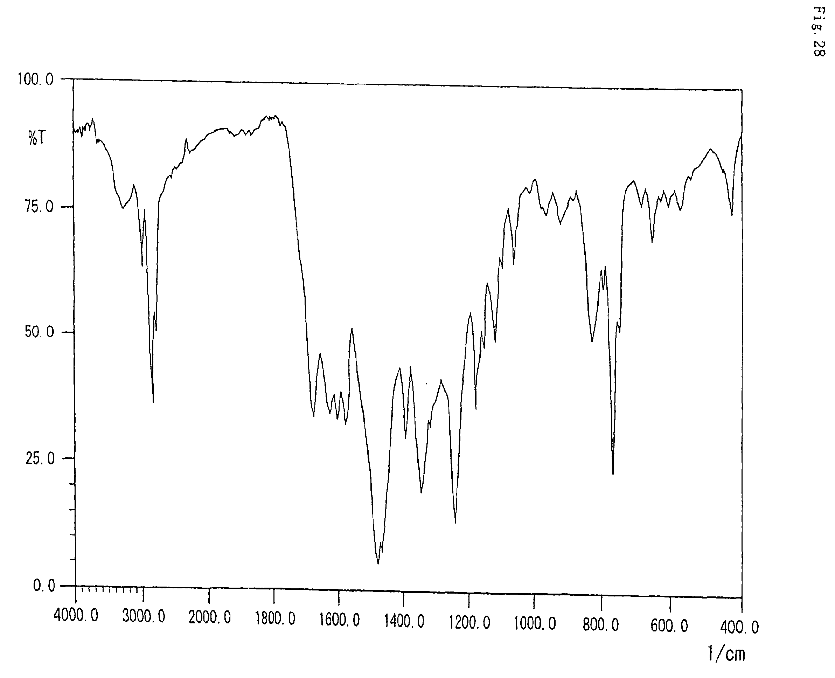

In a 500 ml pressure bottle, 5.0 g (8.0 x 10⁻³ mol) of the compound that had the structure represented by formula (12), the same compound as that obtained in Working Example 1 was placed. 12.9 g (4.5 x 10⁻² mol) of hexyl iodide (C₆H₁₃I) and 200 ml of N,N-dimethylformamide (DMF) were added in this order to the compound. The mixture was heated to 150°C in a silicone oil bath and allowed to react for 20 hours. After the completion of the reaction, the bottle was cooled to the room temperature. Then, the reacted mixture was concentrated with an evaporator. The concentrated mixture was poured into ice water and further neutralized with sodium hydroxide. The neutralized mixture was subjected to extraction with chloroform using a separatory funnel, and the extraction was carried out three times. The extract was washed with water twice and then moisture included therein was removed with sodium sulfate. The washed and dried was filtered, and concentrated and dried up with an evaporator. The obtained solid was washed with petroleum ether, and dried in a vacuum. Liver brown powder was obtained. An IR chart of the liver brown powder is shown in Figure 28.

### <Ring Closing Reaction>

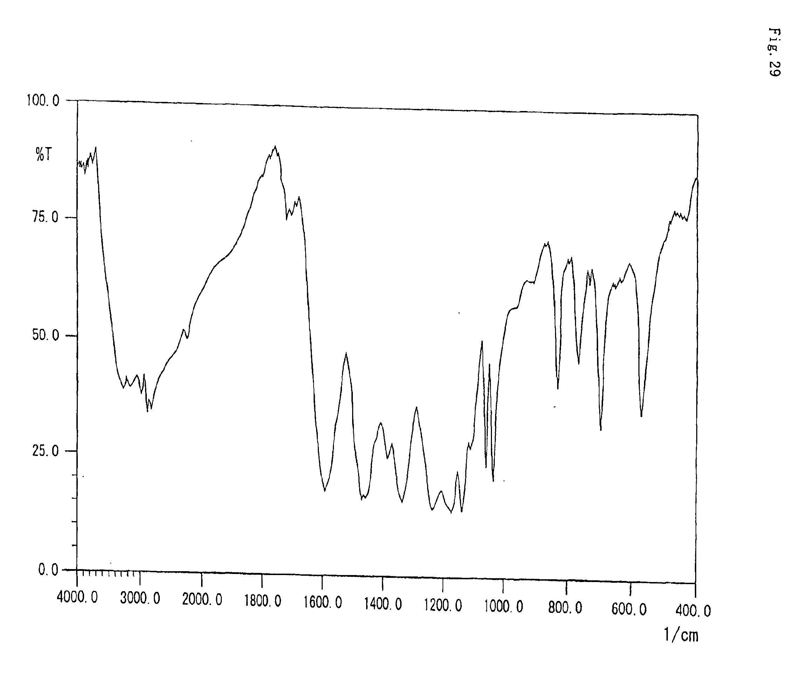

In a 500 ml three-necked flask were placed 5.0 g (5.8 x 10⁻³ mol) of the product obtained in the preceding hexylation reaction, 6.6 g (3.5 x 10⁻² mol) of p-toluenesulfonic acid monohydrate, and 200 ml of o-dichlorobenzene. The mixture was placed in a silicone oil bath and heated to 160°C with stirring. The reaction was allowed to continue for 20 hours. After the termination of the reaction, the reacted mixture was concentrated and dried up with an evaporator. The obtained solid was washed with methyl alcohol that had been cooled to 5 °C, acetone, and petroleum ether in this order, and then vacuum dried. Brown powder was obtained. An IR chart of this brown powder is shown in Figure 29.

### <Purification>

1.0 g of the brown powder obtained in the ring closing reaction in 250 ml of xylene, which was heated to 185°C using a silicone oil bath, was extracted for 24 hours with a Soxhlet abstractor. After the completion of the extraction, the extract was concentrated and dried up with an evaporator. The dried solid was washed with petroleum ether, and dried in a vacuum. Light brown powder was obtained.

### (Working Example 12)

### <Benzylation Reaction>

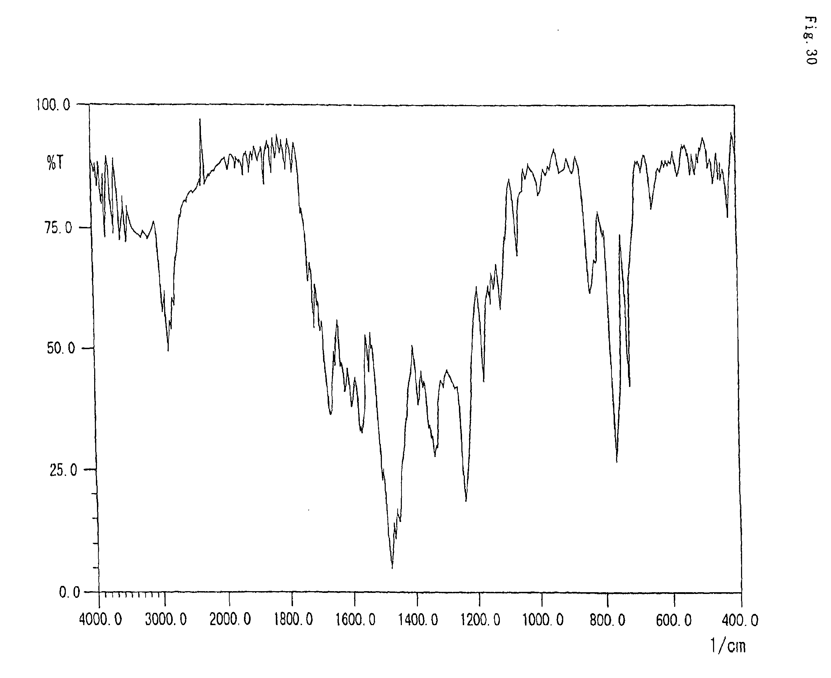

In a 500 ml pressure bottle, 10.0 g (1.6 x 10⁻² mol) of the compound that had the structure represented by formula (12), the same compound as that obtained in Working Example 1 was placed. 17.1 g (7.6 x 10⁻² mol) of benzyl bromide (C₆H₅CH₂Br) and 200 ml of N,N-dimethylformamide (DMF) were added in this order to the compound. The mixture was heated to 150°C in a silicone oil bath and allowed to react for 20 hours. After the completion of the reaction, the bottle was cooled to the room temperature. Then, the reacted mixture was concentrated with an evaporator. The concentrated mixture was poured into ice water and further neutralized with sodium hydroxide. The neutralized mixture was subjected to extraction with chloroform using a separatory funnel, and the extraction was carried out three times. The extract was washed with water twice and then moisture included therein was removed with sodium sulfate. The washed and dried was filtered, and concentrated and dried up with an evaporator. The obtained solid was washed with petroleum ether, and dried in a vacuum. Liver brown powder was obtained. An IR chart of the liver brown powder is shown in Figure 30.

### <Ring Closing Reaction>

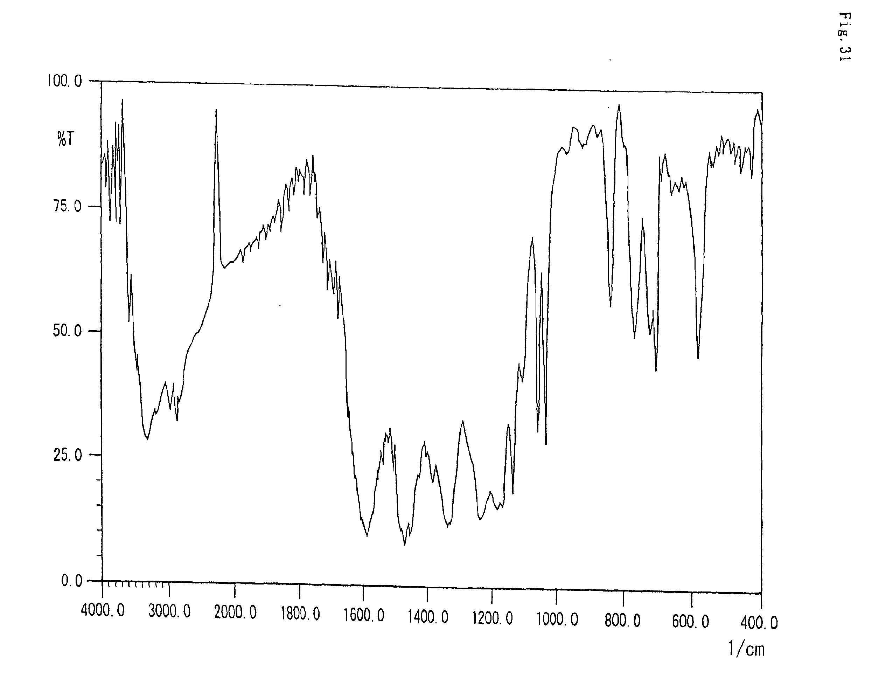

In a 500 ml three-necked flask were placed 5.0 g (6.9 x 10⁻³ mol) of the product obtained in the preceding benzylation reaction, 5.2 g (2.7 x 10⁻² mol) of p-toluenesulfonic acid monohydrate, and 200 ml of o-dichlorobenzene. The mixture was placed in a silicone oil bath and heated to 160°C with stirring. The reaction was allowed to continue for 20 hours. After the termination of the reaction, the reacted mixture was concentrated and dried up with an evaporator. The obtained solid was washed with methyl alcohol that had been cooled to 5°C, acetone, and petroleum ether in this order, and then vacuum dried. Brown powder was obtained. An IR chart of this brown powder is shown in Figure 31.

### (Working Example 13)

### <Dimethylbenzylation, or xylylation Reaction>

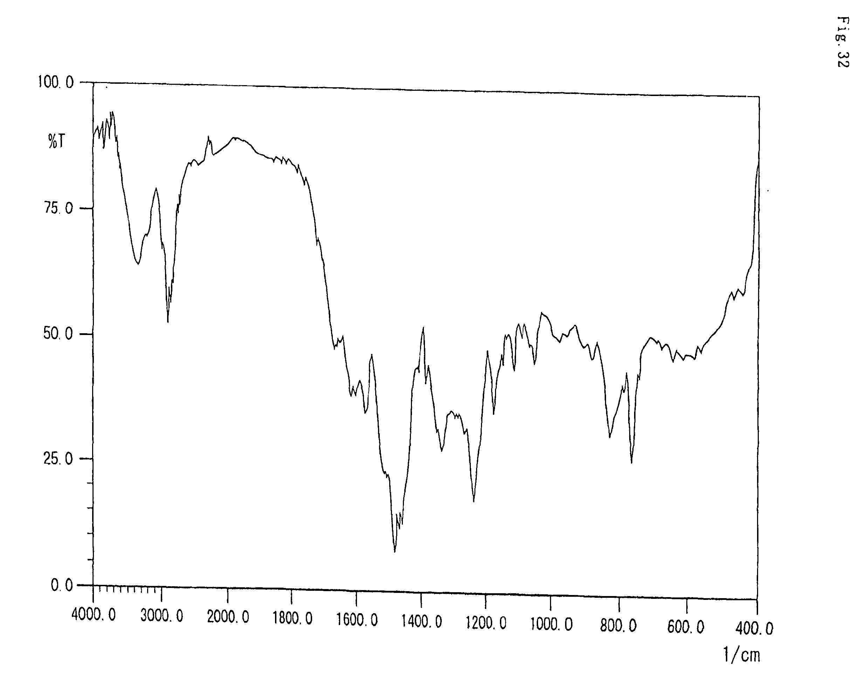

In a 500 ml pressure bottle, 10.0 g (1.6 x 10⁻² mol) of the compound that had the structure represented by formula (12), the same compound as that obtained in Working Example 1 was placed. 15.8 g (1. 0 x 10⁻¹ mol) of 2,4-dimethylbenzyl chloride (CH₃C₆H₃CH₃CH₂Cl) and 200 ml of N,N-dimethylformamide (DMF) were added in this order to the compound. The mixture was heated to 150°C in a silicone oil bath and allowed to react for 20 hours. After the completion of the reaction, the bottle was cooled to the room temperature. Then, the reacted mixture was concentrated with an evaporator. The concentrated mixture was poured into ice water and further neutralized with sodium hydroxide. The neutralized mixture was subjected to extraction with chloroform using a separatory funnel, and the extraction was carried out three times. The extract was washed with water twice and then moisture included therein was removed with sodium sulfate. The washed and dried was filtered, and concentrated and dried up with an evaporator. The obtained solid was washed with petroleum ether, and dried in a vacuum. Liver brown powder was obtained. An IR chart of the liver brown powder is shown in Figure 32.

### <Ring Closing Reaction>

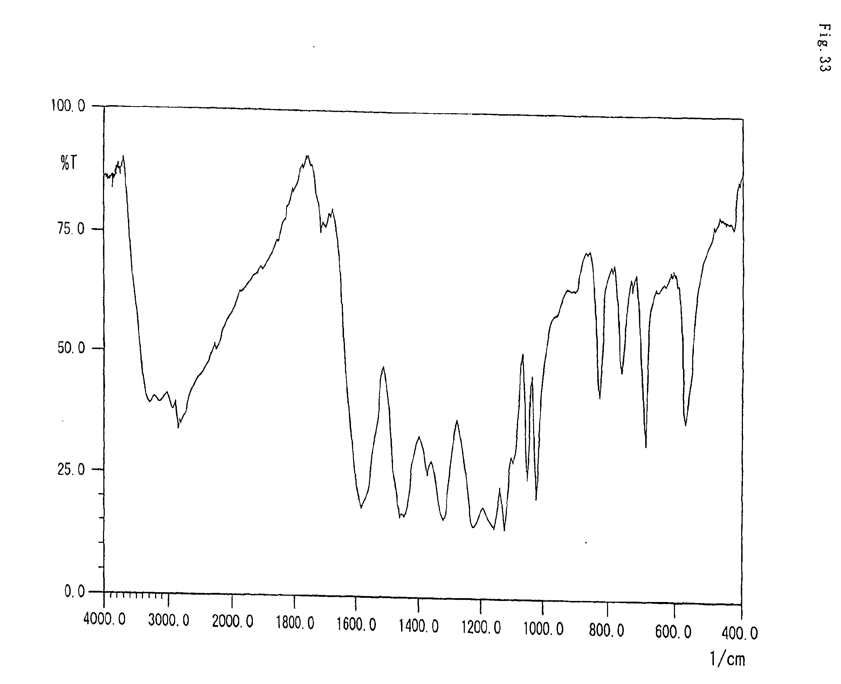

In a 500 ml three-necked flask were placed 5.0 g (6.9 x 10⁻³ mol) of the product obtained in the preceding dimethylbenzylation reaction, 6.0 g (3.2 x 10⁻² mol) of p-toluenesulfonic acid monohydrate, and 200 ml of o-dichlorobenzene. The mixture was placed in a silicone oil bath and heated to 160°C with stirring. The reaction was allowed to continue for 20 hours. After the termination of the reaction, the reacted mixture was concentrated and dried up with an evaporator. The obtained solid was washed with methyl alcohol that had been cooled to 5°C, acetone and petroleum ether in this order, and then vacuum dried. Brown powder was obtained. An IR chart of this brown powder is shown in Figure 33.

### <Purification>

1.0 g of the brown powder obtained in the ring closing reaction in 250 ml of xylene, which was heated to 185°C using a silicone oil bath, was extracted for 24 hours with a Soxhlet abstractor. After the completion of the extraction, the extract was concentrated to dryness with an evaporator. The dried solid was washed with petroleum ether, and dried in a vacuum. Light brown powder was obtained.

### (Working Example 14)

### <Naphthylation Reaction>

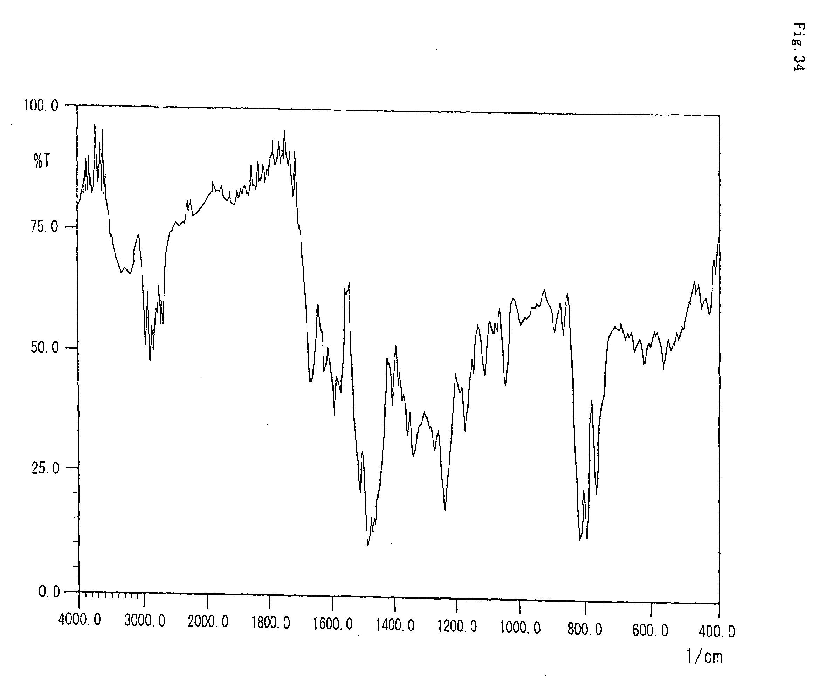

In a 500 ml pressure bottle, 5.0 g (8.0 x 10⁻³ mol) of the compound that had the structure represented by formula (12), the same compound as that obtained in Working Example 1 was placed. 9.6 g (5.4 x 10⁻² mol) of 1-chloromethylnaphthalene (C₁₀H₇CH₂Cl) and 200 ml of N, N-dimethylformamide (DMF) were added in this order to the compound. The mixture was heated to 150°C in a silicone oil bath and allowed to react for 20 hours. After the completion of the reaction, the bottle was cooled to the room temperature. Then, the reacted mixture was concentrated with an evaporator. The concentrated mixture was poured into ice water and further neutralized with sodium hydroxide. The neutralized mixture was subjected to extraction with chloroform using a separatory funnel, and the extraction was carried out three times. The extract was washed with water twice, and then water included therein was removed with sodium sulfate. The washed and dried was filtered, concentrated to dryness with an evaporator. The obtained solid was washed with petroleum ether, and dried in a vacuum. Liver brown powder was obtained. An IR chart of the liver brown powder is shown in Figure 34.

### <Ring Closing Reaction>

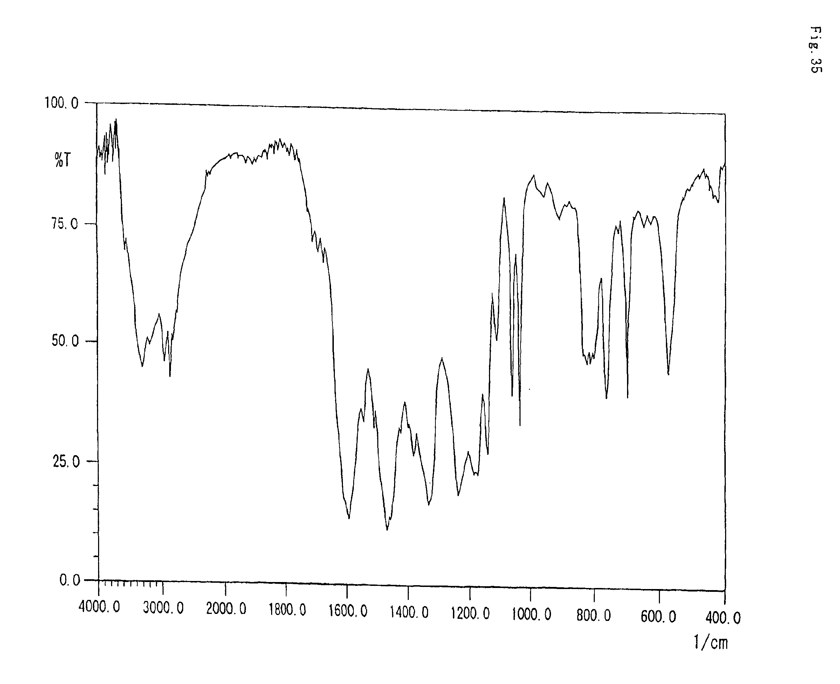

In a 500 ml three-necked flask were placed 5.0 g (6.0 x 10⁻³ mol) of the product obtained in the preceding naphthylation reaction, 6.9 g (3.6 x 10⁻² mol) of p-toluenesulfonic acid monohydrate, and 200 ml of o-dichlorobenzene. The mixture was placed in a silicone oil bath and heated to 160°C with stirring. The reaction was allowed to continue for 20 hours. After the termination of the reaction, the reacted mixture was concentrated and dried up with an evaporator. The obtained solid was washed with methyl alcohol that had been cooled to 5°C, acetone, and petroleum ether in this order, and then vacuum dried. Brown powder was obtained. An IR chart of this brown powder is shown in Figure 35.

### <Purification>

1.0 g of the brown powder obtained in the ring closing reaction in 250 ml of xylene, which was heated to 185°C using a silicone oil bath, was extracted for 24 hours with a Soxhlet abstractor. After the completion of the extraction, the extract was concentrated and dried up with an evaporator. The dried solid was washed with petroleum ether, and dried in a vacuum. Liver brown powder was obtained.

### (Working Example 15)

### <Anthrylation Reaction>

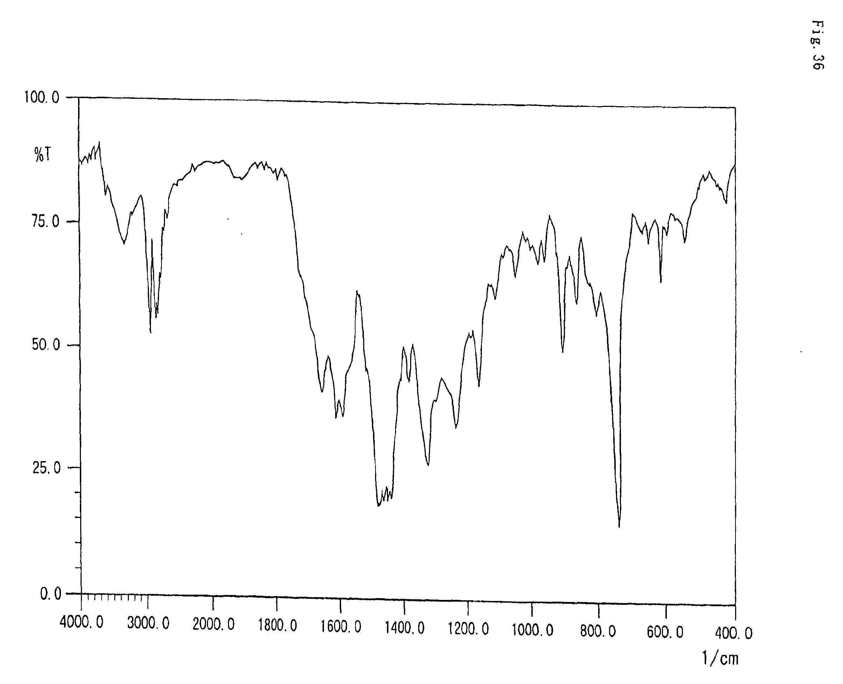

In a 500 ml pressure bottle, 5.0 g (8.0 x 10⁻³ mol) of the compound that had the structure represented by formula (12), the same compound as that obtained in Working Example 1 was placed. 8.6 g (5.4 x 10⁻² mol) of 9-chloromethylanthracene (C₁₄H₇CH₂Cl) and 200 ml of N,N-dimethylformamide (DMF) were added in this order to the compound. The mixture was heated to 150°C in a silicone oil bath and allowed to react for 20 hours. After the completion of the reaction, the bottle was cooled to the room temperature. Then, the reacted mixture was concentrated with an evaporator. The concentrated mixture was poured into ice water and further neutralized with sodium hydroxide. The neutralized mixture was subjected to extraction with chloroform using a separatory funnel, and the extraction was repeated twice more. The extract was washed with water twice, and then moisture included therein was removed with sodium sulfate. The washed and dried was filtered, and concentrated and dried up with an evaporator. The obtained solid was washed with petroleum ether, and dried in a vacuum. Liver brown powder was obtained. An IR chart of the liver brown powder is shown in Figure 36.

### <Ring Closing Reaction>

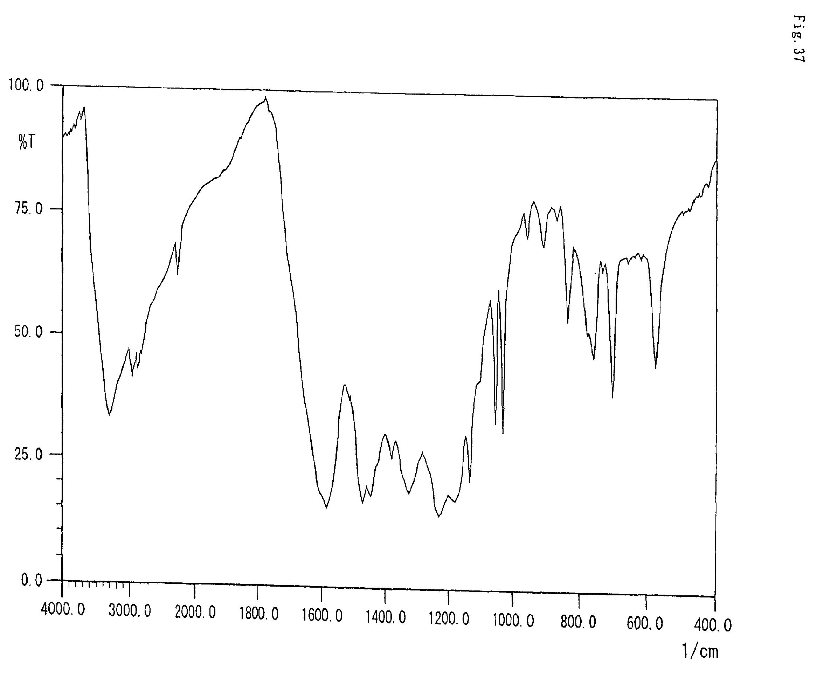

In a 500 ml three-necked flask were placed 5.0 g (5.3 x 10⁻³ mol) of the product obtained in the preceding anthrylation reaction, 5.1 g (2.6 x 10⁻² mol) of p-toluenesulfonic acid monohydrate, and 200 ml of o-dichlorobenzene. The mixture was placed in a silicone oil bath and heated to 160°C with stirring. The reaction was allowed to continue for 20 hours. After the termination of the reaction, the reacted mixture was concentrated and dried up with an evaporator. The obtained solid was washed with methyl alcohol that had been cooled to 5 °C, acetone, and petroleum ether in this order, and then vacuum dried. Brown powder was obtained. An IR chart of this brown powder is shown in Figure 37.

### <Purification>

1.0 g of the brown powder obtained in the ring closing reaction in 250 ml of xylene, which was heated to 185°C using a silicone oil bath, was extracted for 24 hours with a Soxhlet abstractor. After the completion of the extraction, the extract was concentrated and dried up with an evaporator. The dried solid was washed with petroleum ether, and dried in a vacuum. Light brown powder was obtained.

### (Working Example 16)

### <Substituted-methylbenzylation Reaction>

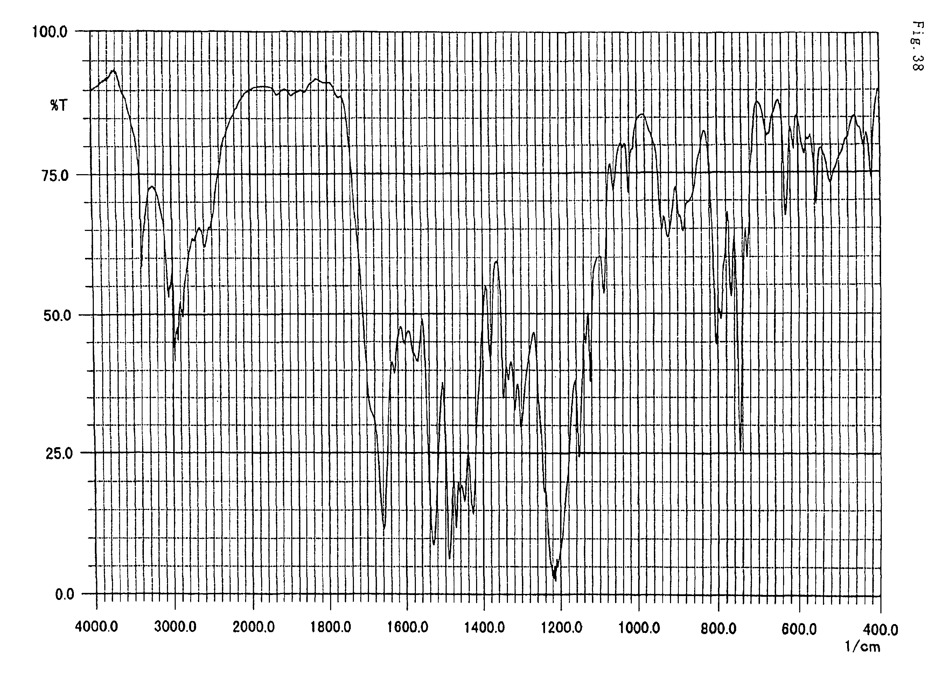

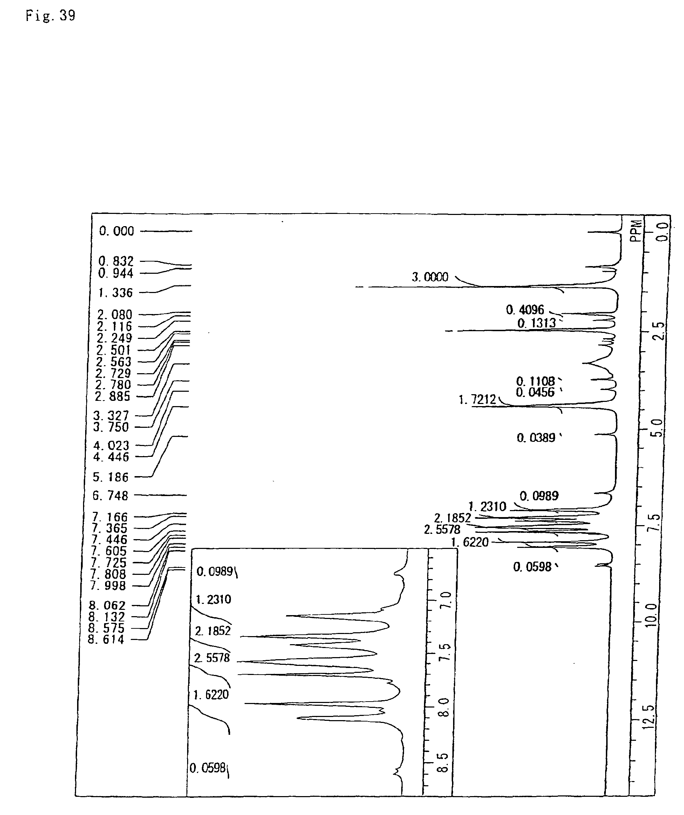

In a 1,000 ml pressure bottle, 10.0 g (1.6 x 10⁻² mol) of the compound that had the structure represented by formula (12), the same compound as that obtained in Working Example 1 was placed. 2.3 g (1.6 x 10⁻² mol), the number of moles of which was the same as that of the compound (12), of α-chloro-p-xylene and 500 ml of N, N-dimethylformamide (DMF) were added in this order to the compound. The mixture was heated to 150°C in a silicone oil bath and allowed to react for 20 hours. After the completion of the reaction, the bottle was cooled to the room temperature. Then, the reacted mixture was concentrated with an evaporator. The concentrated mixture was poured into ice water and further neutralized with sodium hydroxide. The neutralized mixture was subjected to extraction with chloroform using a separatory funnel, and the extraction was repeated twice more. The extract was washed with water twice, and then moisture included therein was removed with sodium sulfate. The washed and dried was filtered, and concentrated and dried up with an evaporator. The obtained solid was washed with petroleum ether, and dried in a vacuum. Blue brown powder was obtained. An IR chart of the blue brown powder is shown in Figure 38, and an NMR chart thereof in Figure 39.

### <Ring Closing Reaction>

In a 500 ml three-necked flask were placed 5.0 g (7.0 x 10⁻³ mol) of the product obtained in the preceding naphthylation reaction, 8.0 g (4.2 x 10⁻² mol) of p-toluenesulfonic acid monohydrate, and 200 ml of o-dichlorobenzene. The mixture was placed in a silicone oil bath and heated to 160°C with stirring. The reaction was allowed to continue for 20 hours. After the termination of the reaction, the reacted mixture was concentrated and dried up with an evaporator. The obtained solid was washed with methyl alcohol that had been cooled to 5 °C, acetone, and petroleum ether in this order, and then vacuum dried. Brown solid was obtained.

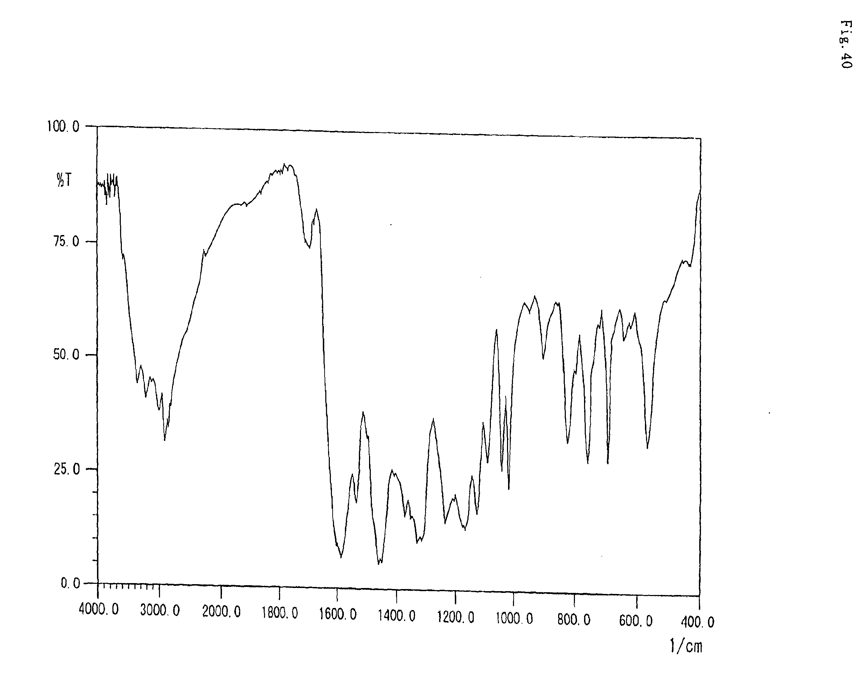

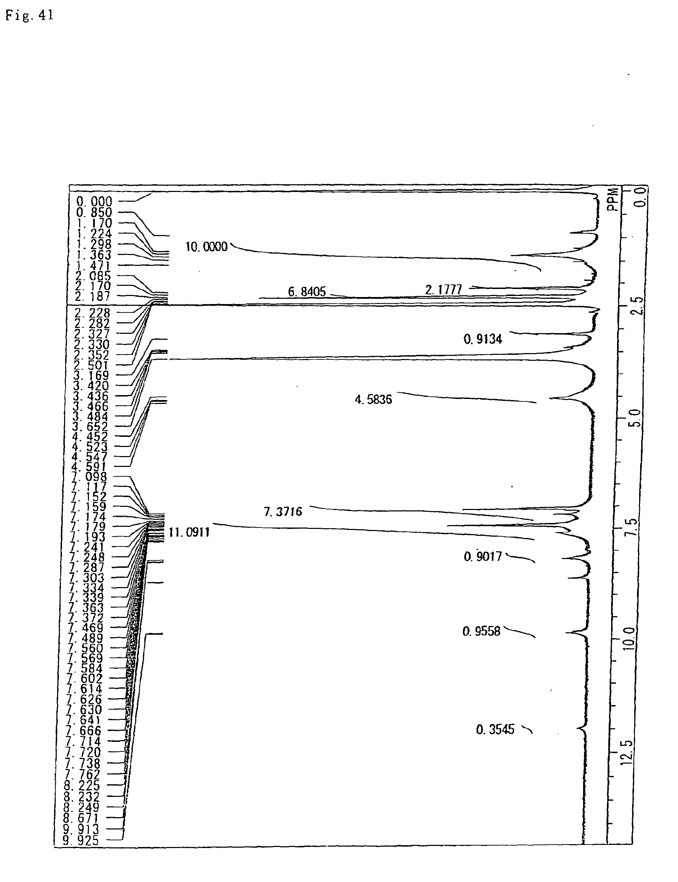

1.0 g of the obtained brown solid in 250 ml of xylene, which was heated to 185°C using a silicone oil bath, was extracted for 24 hours with a Soxhlet abstractor. After the completion of the extraction, the extract was concentrated and dried up with an evaporator. The dried solid was washed with petroleum ether, and dried in a vacuum. Light brown powder was obtained. An IR chart of the light brown powder is shown in Figure 40, and an NMR chart thereof in Figure 41. This light brown powder was identified as a compound that had the structure represented by formula (1e) below.

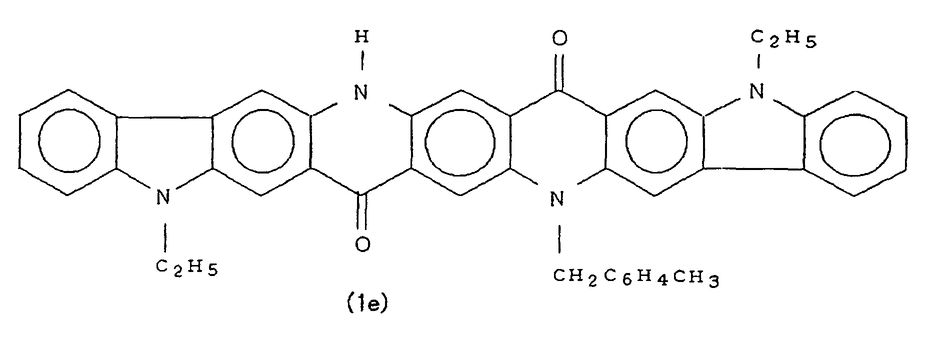

### <Luminescent Properties>

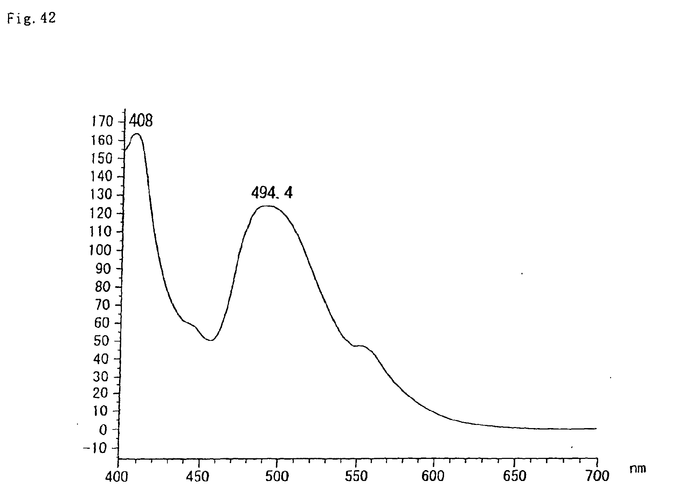

A sample solution was prepared by dissolving the white light-emitting compound represented by formula (1e) in mixed xylene, so that the concentration of the compound was 10 mg/L. This sample solution was loaded in a model F-4500 spectrofluorophotometer, a product by Shimadzu Corporation, and the fluorescence spectrum of the solution was measured under the following conditions. The measured spectrum is shown in Figure 42.

### Conditions of Measurement

Measuring mode: Wavelength scanning
Exciting wavelength: 365 nm
Wavelength at which the emission of fluorescence started: 400 nm
Wavelength at which the emission of fluorescence ended: 700 nm
Scanning speed: 1200 nm/min.
Slit on the side of excitation: 5.0 nm
Slit on the side of fluorescence emission: 5.0 nm
Photomal voltage: 700 V

As understood from Figure 42, the fluorescence of the white light-emitting compound obtained in this example covers wavelengths of from 400 nm to 700 nm.

### Industrial Applicability

This invention can provide a luminescent compound capable of emitting white light by itself. The emission covers a visible light range of 400-700 nm, and the luminance is not less than 2,000 Cd/m². The invention can also provide an illuminator and organic EL element capable of emitting white light by the employment of this compound. The illuminator or element may be used for displays, illuminating devices, etc. that emit white light. Also, it may be applied to advertising apparatuses that are on in the dark, and road traffic signs.

## Claims

1. A luminescent compound capable of emitting white light that has a structure represented by formula (1): 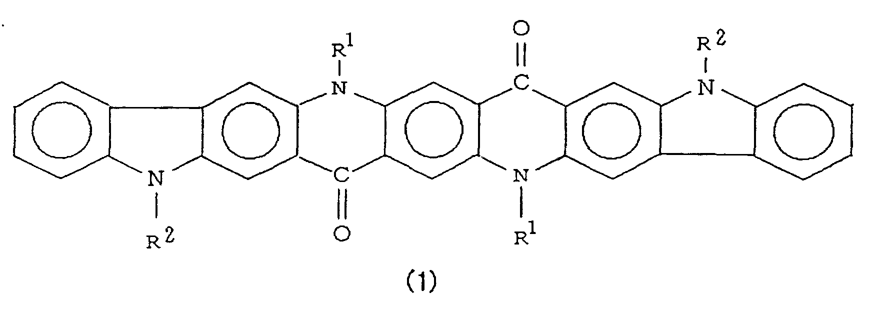 wherein R¹ is hydrogen atom, an alkyl group, or an aryl or alkyl aryl group that may have at least one substituent, wherein two R¹s may be the same or different from each other; R² is hydrogen atom, an alkyl group, or an aryl or alkyl aryl group that may have at least one substituent, wherein two R²s may be the same or different from each other; and R¹ and R² may be the same or different from each other.

2. A luminescent compound capable of emitting white light that has a structure represented by formula (2): 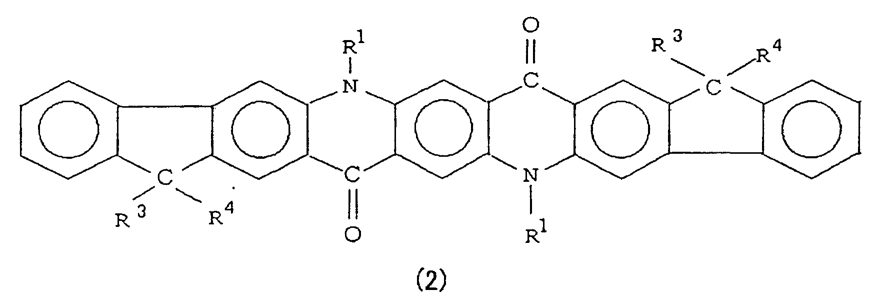 wherein R¹ is hydrogen atom, an alkyl group, or an aryl or alkyl aryl group that may have at least one substituent, wherein two R¹s may be the same or different from each other; each of R³ and R⁴ is hydrogen atom, an alkyl group, or an aryl or alkyl aryl group that may have at least one substituent, wherein R³ and R⁴ may be the same or different from each other; and two R³s may be the same or different and two R⁴s may be the same or different.

3. An illuminator capable of emitting white light comprising a substrate on which an electrode has been formed, and a light emitting layer which is placed on the substrate, said layer including at least one light-emitting material selected from the group consisting of said compound represented by formula (1) and said compound represented by formula (2), wherein each of said compounds emits white light by itself.

4. The illuminator as claimed in claim 3, wherein said illuminator, which comprises the substrate and the light emitting layer placed on the substrate, said layer including at least one light-emitting material selected from the group consisting of said compound represented by formula (1) and said compound represented by formula (2), is a one-layer type organic EL element.

5. The illuminator as claimed in claim 3, wherein said illuminator is a multi-layer type organic EL element further comprising a hole-transporting layer and an electron-transporting layer.

6. The illuminator as claimed in any one of claims 3-5, wherein said light-emitting layer is a layer prepared by dispersing the compound in a high polymer.

7. The illuminator as claimed in any one of claims 3-5, wherein said light-emitting layer is a layer prepared by depositing said compound on said substrate.

8. The illuminator as claimed in any one of claims 3-7, wherein said illuminator is a planar illuminator or a tubular illuminator.

9. An organic EL element comprising a substrate on which an electrode has been formed, and a light-emitting layer formed on said substrate, said light-emitting layer including at least one luminescent material capable of emitting white light selected from the group consisting of the compound represented by formula (1) and the compound represented by formula (2).
